# EUROPEAN PATENT SPECIFICATION

(11) **EP 4 125 560 B1**
(45) Date of publication and mention of the grant of the patent: **19.08.2026**
(21) Application number: 21722544.0
(22) Date of filing: 31.03.2021
(51) Int. Cl.: A61B 5/00, A61M 16/08, A61B 5/11, A61B 5/113, H05B 3/00

(54) **SYSTEMS AND METHODS FOR DETERMINING MOVEMENT OF A CONDUIT**
SYSTEME UND VERFAHREN ZUR BESTIMMUNG DER BEWEGUNG EINER LEITUNG
SYSTÈMES ET PROCÉDÉS DE DÉTERMINATION DE MOUVEMENT D'UNE CONDUITE

(30) Priority: 31.03.2020 US 202063003110 P
(43) Date of publication of application: 08.02.2023
(73) Proprietor: ResMed Sensor Technologies Limited, Dublin, D18 T6T7 (IE)
(72) Inventor: FOX, Niall, Dublin, D18 T6T7 (IE); MCMAHON, Stephen, Dublin, D18 T6T7 (IE); SHOULDICE, Redmond, Dublin, D18 T6T7 (IE)
(74) Representative: Mathys & Squire
(86) International application number: PCT/IB2021/052671
(87) International publication number: WO 2021/198935

(56) References cited:
- WO-A1-2017/132726
- US-A- 2 811 855
- US-A1- 2011 023 874

## Description

### CROSS-REFERENCE TO RELATED APPLICATIONS

This application claims the benefit of, and priority to, U.S. Provisional Patent Application No. 63/003,110 filed on March 31, 2020.

### TECHNICAL FIELD

The present disclosure relates generally to systems and methods for determining movement of a conduit, and more particularly, to systems and methods for determining movement of a conduit based at least in part on changes in an electrical property of an electronic circuit in the conduit.

### BACKGROUND

Various systems exist for aiding users experiencing sleep and related respiratory disorders. A range of respiratory disorders exist that can impact users. Certain disorders are characterized by particular events (e.g., apneas, hypopneas, hyperpneas, or any combination thereof). Examples of sleep- and respiratory-related disorders include periodic limb movement disorder (PLMD), Obstructive Sleep Apnea (OSA), Cheyne-Stokes Respiration (CSR), respiratory insufficiency, Obesity Hyperventilation Syndrome (OHS), Chronic Obstructive Pulmonary Disease (COPD), Neuromuscular Disease (NMD), and Chest wall disorders. When a user is undergoing respiratory therapy, movement information associated with the user can be used for, for example, sleep-wake determination. Thus, a need exists for systems and methods for determining movement associated with users undergoing respiratory therapy. The present disclosure is directed to solving these and other problems.

### SUMMARY

According to some implementations, a method for determining movement of a conduit is disclosed. Data associated with a first electrical property of a portion of an electronic circuit is received. The electronic circuit is coupled to the conduit. The first electrical property is configured to change based at least in part on movement of the portion of the electronic circuit. The received data is analyzed. Based at least in part on the analysis, it is determined that the first electrical property of the electronic circuit has changed. Responsive to the determination that the first electrical property of the electronic circuit has changed, it is determined that the conduit is moving or has moved.

According to some implementations of the present disclosure, a system includes an electronic circuit, a memory, and a control system. The electronic circuit is coupled to a conduit. The conduit may be configured to deliver pressurized air. A portion of the electronic circuit has a first electrical property that is configured to change based at least in part on movement of the portion of the electronic circuit. The memory stores machine-readable instructions. The control system includes one or more processors configured to execute the machine-readable instructions. Data associated with the first electrical property of the electronic circuit is received. The received data is analyzed. Based at least in part on the analysis, it is determined that the first electrical property of the electronic circuit has changed. Responsive to the determination that the first electrical property of the electronic circuit has changed, it is determined that the conduit is moving or has moved.

According to some implementations of the present disclosure, a system includes a first electronic circuit and a second electronic circuit. The first electronic circuit is coupled to a conduit. The conduit may be configured to deliver pressurized air. A first segment of the first electronic circuit, which corresponds to a first segment of the conduit, has an electrical resistance that is configured to change by at least a first percentage based on movement of at least a portion of the first segment of the first electronic circuit. A second segment of the first electronic circuit, which corresponds to a second segment of the conduit, has an electrical resistance that is configured to change no more than a second percentage that is less than the first percentage based on movement of the second segment of the first electronic circuit. The second electronic circuit is coupled to the conduit. A first segment of the second electronic circuit, which corresponds to the first portion of the conduit, has an electrical resistance that is configured to change no more than the second percentage based on movement of the first segment of the second electronic circuit. A second segment of the second electronic circuit, which corresponds to the second segment of the conduit, has an electrical resistance that is configured to change by at least the first percentage based on movement of at least a portion of the second segment of the second electronic circuit.

According to some implementations of the present disclosure, a method for determining movement of a conduit is disclosed. First data associated with a first electrical resistance of a first electronic circuit is received. The first electronic circuit is coupled to the conduit. A first segment of the first electronic circuit, which corresponds to a first segment of the conduit, has an electrical resistance that is configured to change by at least a first percentage based on movement of at least a portion of the first segment of the first electronic circuit. A second segment of the first electronic circuit, which corresponds to a second segment of the conduit, has an electrical resistance that is configured to change no more than a second percentage that is less than the first percentage based on movement of the second segment of the first electronic circuit. Second data associated with a second electrical resistance of a second electronic circuit is received. The second electronic circuit is coupled to the conduit. A first segment of the second electronic circuit, which corresponds to the first portion of the conduit, has an electrical resistance that is configured to change no more than the second percentage based on movement of the first segment of the second electronic circuit. A second segment of the second electronic circuit, which corresponds to the second segment of the conduit, has an electrical resistance that is configured to change by at least the first percentage based on movement of at least a portion of the second segment of the second electronic circuit. The received first data and the received second data are analyzed to determine if (i) the first electrical resistance of the first electronic circuit has changed, (ii) the second electrical resistance of the second electronic circuit has changed, or (iii) both (i) and (ii). Responsive to a determination that the first electrical resistance of the first electronic circuit has changed, it is determined that the first portion of the conduit is moving or has moved. Responsive to a determination that the second electrical resistance of the second electronic circuit has changed, it is determined that the second portion of the conduit is moving or has moved.

The above summary is not intended to represent each implementation or every aspect of the present disclosure. Additional features and benefits of the present disclosure are apparent from the detailed description and figures set forth below.

### BRIEF DESCRIPTION OF THE DRAWINGS

FIG. 1 is a functional block diagram of a system, according to some implementations of the present disclosure;
FIG. 2 is a perspective view of at least a portion of the system of FIG. 1, a user, and a bed partner, according to some implementations of the present disclosure;
FIG. 3 depicts an electronic circuit including a first electronic component having a first electrical property that is configured to change based on movement of the first electronic component, according to some implementations of the present disclosure;
FIG. 4 depicts an electronic circuit including a first portion having a first electrical property that is configured to change based on movement of the first portion, according to some implementations of the present disclosure;
FIG. 5 depicts the first electronic component of FIG. 3 coupled to a heating wire, according to some implementations of the present disclosure; and
FIG. 6 depicts example movement data and temperature data on a frequency-amplitude plot, according to some implementations of the present disclosure.

While the present disclosure is susceptible to various modifications and alternative forms, specific implementations and embodiments thereof have been shown by way of example in the drawings and will herein be described in detail. It should be understood, however, that it is not intended to limit the present disclosure to the particular forms disclosed. The invention is defined by the appended claims.

### DETAILED DESCRIPTION

Respiratory pressure therapy devices (referred to herein as respiratory devices) can be used to aid users experiencing respiratory disorders. Respiratory devices are unhelpful and unable to provide therapeutic relief if users do not actually use the respiratory devices while sleeping. There is a need to monitor users of respiratory devices to carry out tasks such as sleep-staging. When users are using respiratory therapy, the flow signal can be used to determine respiration statistics, which in turn can be used to determine the sleep state and/or sleep stage. A challenge with only having respiration information from the flow signal, is that it can be difficult to accurately distinguish between sleep and wake for a user of the respiratory device.

As disclosed herein, movement information associated with a user of the respiratory device can be used for sleep-wake determination. For example, theoretically, if the user is moving, the determination can be that the user is awake; and if the user is not moving, the determination can be that the user asleep. However, practically, determining sleep-related activities (e.g., bruxism, sleep state, sleep stage, body position, head position, etc.) from movement information is not that simple, because the user can still move when asleep, such as rolling over, twitching legs, etc. Thus, differentiating between movements that occur when the user is awake, versus movements that occur when the user is asleep, requires additional analysis of the movement information.

In some implementations of the present disclosure, the analysis to determine the sleep-related activities can include analyzing the frequency of movement and/or the accumulation of movement overtime. For example, shorter duration and/or more sporadic movement may indicate that the user is less likely to be awake. In some implementations, the analysis to determine the sleep-related activities can include analyzing body temperature of the user and/or ambient temperature associated with the user. For example, the user's core body temperature changes during different sleep phases. In addition, the user's peripheral temperature also changes due to blood flow, which varies during different sleep phases.

However, movement information is poorly captured by the flow signal alone. Even though other sensors are also contemplated to be used to generate movement information, such as: RF sensor, accelerometer on the user interface, microphone in the respiratory device (via direct acoustic measurement), and wearable (e.g., wrist-based) sensor, the present disclosure relates to systems and methods for determining movement of the conduit, which is indicative of movement of the user associated with the conduit.

According to some implementations, the conduit of the respiratory therapy system includes a tube (e.g., a plastic tube) with a coiled resistance wire embed within. The tube is heated by passing an electric current through the coiled resistance wire. The tube is electrically connected to a flow generator in the respiratory device for heating the tube. Variations in tube topology results in variations in resistance of the coiled resistance wire, which could be measured to provide an indication of movement of the conduit. The additional cost of this implementation is minimal as the tube could be used "as is." For example, in some such implementations, only a simple heater circuit current sensor is added to the flow generator to measure these variations in resistance.

In some implementations, a movement sensor of the conduit can be itself a metal wire. The metal wire can be straight or coiled. The variations in resistance, over time, can indicate movement of the conduit. In some implementations, initial data can be used as test data for determining a threshold change associated with actual movement of the conduit. For example, moving the conduit 126 from a first position and/or orientation to a second position and/or orientation can result in a delta (e.g., difference) between the measured resistances in the conduit. Furthermore, machine learning can also be implemented to associate variations in resistance to sleep-related activities (e.g., bruxism, sleep state, sleep stage, body position, head position, etc.). For example, machine learning can be used to correlate measured current data (e.g., indicative of variations in resistance) with measured physiological data from other sensors.

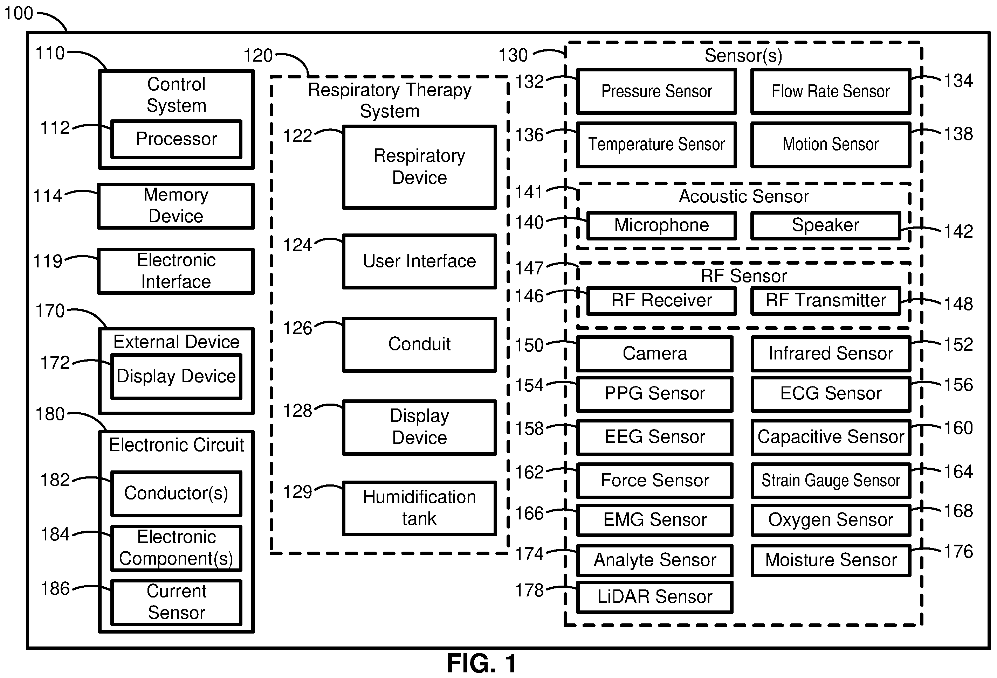

Referring to FIG. 1, a system 100, according to some implementations of the present disclosure, is illustrated. The system 100 includes a control system 110, a memory device 114, an electronic interface 119, one or more sensors 130, and one or more external devices 170. In some implementations, the system 100 further optionally includes a respiratory therapy system 120.

The control system 110 includes one or more processors 112 (hereinafter, processor 112). The control system 110 is generally used to control (e.g., actuate) the various components of the system 100 and/or analyze data obtained and/or generated by the components of the system 100. The processor 112 can be a general or special purpose processor or microprocessor. While one processor 112 is illustrated in FIG. 1, the control system 110 can include any number of processors (e.g., one processor, two processors, five processors, ten processors, etc.) that can be in a single housing, or located remotely from each other. The control system 110 (or any other control system) or a portion of the control system 110 such as the processor 112 (or any other processor(s) or portion(s) of any other control system), can be used to carry out one or more steps of any of the methods described and/or claimed herein. The control system 110 can be coupled to and/or positioned within, for example, a housing of the external device 170, a portion (e.g., a housing) of the respiratory therapy system 120, and/or within a housing of one or more of the sensors 130. The control system 110 can be centralized (within one such housing) or decentralized (within two or more of such housings, which are physically distinct). In such implementations including two or more housings containing the control system 110, such housings can be located proximately and/or remotely from each other.

The memory device 114 stores machine-readable instructions that are executable by the processor 112 of the control system 110. The memory device 114 can be any suitable computer readable storage device or media, such as, for example, a random or serial access memory device, a hard drive, a solid state drive, a flash memory device, etc. While one memory device 114 is shown in FIG. 1, the system 100 can include any suitable number of memory devices 114 (e.g., one memory device, two memory devices, five memory devices, ten memory devices, etc.). The memory device 114 can be coupled to and/or positioned within a housing of a respiratory device 122 of the respiratory therapy system 120, within a housing of the external device 170, within a housing of one or more of the sensors 130, or any combination thereof. Like the control system 110, the memory device 114 can be centralized (within one such housing) or decentralized (within two or more of such housings, which are physically distinct).

In some implementations, the memory device 114 stores a user profile associated with the user. The user profile can include, for example, demographic information associated with the user, biometric information associated with the user, medical information associated with the user, self-reported user feedback, sleep parameters associated with the user (e.g., sleep-related parameters recorded from one or more earlier sleep sessions), or any combination thereof. The demographic information can include, for example, information indicative of an age of the user, a gender of the user, a race of the user, a geographic location of the user, a relationship status, a family history of insomnia or sleep apnea, an employment status of the user, an educational status of the user, a socioeconomic status of the user, or any combination thereof. The medical information can include, for example, information indicative of one or more medical conditions associated with the user, medication usage by the user, or both. The medical information data can further include a multiple sleep latency test (MSLT) result or score and/or a Pittsburgh Sleep Quality Index (PSQI) score or value. The self-reported user feedback can include information indicative of a self-reported subjective sleep score (e.g., poor, average, excellent), a self-reported subjective stress level of the user, a self-reported subjective fatigue level of the user, a self-reported subjective health status of the user, a recent life event experienced by the user, or any combination thereof.

The electronic interface 119 is configured to receive data (e.g., physiological data and/or audio data) from the one or more sensors 130 such that the data can be stored in the memory device 114 and/or analyzed by the processor 112 of the control system 110. The electronic interface 119 can communicate with the one or more sensors 130 using a wired connection or a wireless connection (e.g., using an RF communication protocol, a Wi-Fi communication protocol, a Bluetooth communication protocol, over a cellular network, etc.). The electronic interface 119 can include an antenna, a receiver (e.g., an RF receiver), a transmitter (e.g., an RF transmitter), a transceiver, or any combination thereof. The electronic interface 119 can also include one more processors and/or one more memory devices that are the same as, or similar to, the processor 112 and the memory device 114 described herein. In some implementations, the electronic interface 119 is coupled to or integrated in the external device 170. In other implementations, the electronic interface 119 is coupled to or integrated (e.g., in a housing) with the control system 110 and/or the memory device 114.

As noted above, in some implementations, the system 100 optionally includes a respiratory therapy system 120. The respiratory therapy system 120 can include a respiratory pressure therapy (RPT) device 122 (referred to herein as respiratory device 122), a user interface 124, a conduit 126 (also referred to as a tube or an air circuit), a display device 128, a humidification tank 129, or any combination thereof. In some implementations, the control system 110, the memory device 114, the display device 128, one or more of the sensors 130, and the humidification tank 129 are part of the respiratory device 122. Respiratory pressure therapy refers to the application of a supply of air to an entrance to a user's airways at a controlled target pressure that is nominally positive with respect to atmosphere throughout the user's breathing cycle (e.g., in contrast to negative pressure therapies such as the tank ventilator or cuirass). The respiratory therapy system 120 is generally used to treat individuals suffering from one or more sleep-related respiratory disorders (e.g., obstructive sleep apnea, central sleep apnea, or mixed sleep apnea).

The respiratory device 122 is generally used to generate pressurized air that is delivered to a user (e.g., using one or more motors that drive one or more compressors). In some implementations, the respiratory device 122 generates continuous constant air pressure that is delivered to the user. In other implementations, the respiratory device 122 generates two or more predetermined pressures (e.g., a first predetermined air pressure and a second predetermined air pressure). In still other implementations, the respiratory device 122 is configured to generate a variety of different air pressures within a predetermined range. For example, the respiratory device 122 can deliver at least about 6 cmH₂O, at least about 10 cmH₂O, at least about 20 cmH₂O, between about 6 cmH₂O and about 10 cm H₂O, between about 7 cmH₂O and about 12 cmH₂O, etc. The respiratory device 122 can also deliver pressurized air at a predetermined flow rate between, for example, about -20 L/min and about 150 L/min, while maintaining a positive pressure (relative to the ambient pressure).

The user interface 124 engages a portion of the user's face and delivers pressurized air from the respiratory device 122 to the user's airway to aid in preventing the airway from narrowing and/or collapsing during sleep. This may also increase the user's oxygen intake during sleep. Generally, the user interface 124 engages the user's face such that the pressurized air is delivered to the user's airway via the user's mouth, the user's nose, or both the user's mouth and nose. Together, the respiratory device 122, the user interface 124, and the conduit 126 form an air pathway fluidly coupled with an airway of the user. The pressurized air also increases the user's oxygen intake during sleep.

Depending upon the therapy to be applied, the user interface 124 may form a seal, for example, with a region or portion of the user's face, to facilitate the delivery of gas at a pressure at sufficient variance with ambient pressure to effect therapy, for example, at a positive pressure of about 10 cmH₂O relative to ambient pressure. For other forms of therapy, such as the delivery of oxygen, the user interface may not include a seal sufficient to facilitate delivery to the airways of a supply of gas at a positive pressure of about 10 cmH₂O.

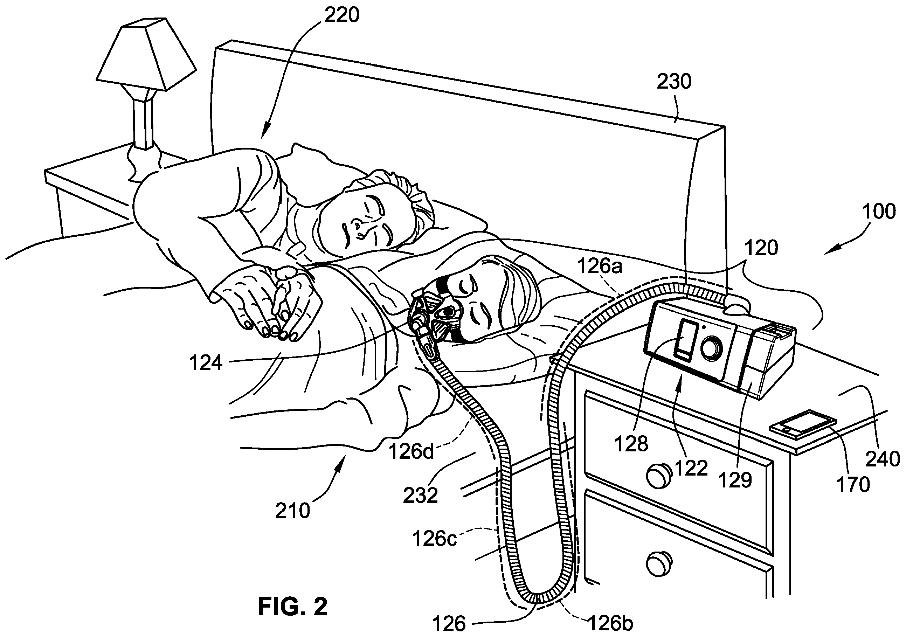

As shown in FIG. 2, in some implementations, the user interface 124 is a facial mask (e.g., a full face mask) that covers the nose and mouth of the user. Alternatively, the user interface 124 can be a nasal mask that provides air to the nose of the user or a nasal pillow mask that delivers air directly to the nostrils of the user. The user interface 124 can include a plurality of straps forming, for example, a headgear for aiding in positioning and/or stabilizing the user interface 124 on a portion of the user (e.g., the face) and a conformal cushion (e.g., silicone, plastic, foam, etc.) that aids in providing an air-tight seal between the user interface 124 and the user. The user interface 124 can also include one or more vents for permitting the escape of carbon dioxide and other gases exhaled by the user 210. In other implementations, the user interface 124 includes a mouthpiece (e.g., a night guard mouthpiece molded to conform to the teeth of the user, a mandibular repositioning device, etc.).

The conduit 126 (also referred to as an air circuit or tube) allows the flow of air between two components of a respiratory therapy system 120, such as the respiratory device 122 and the user interface 124. In some implementations, there can be separate limbs of the conduit for inhalation and exhalation. In other implementations, a single limb conduit is used for both inhalation and exhalation.

One or more of the respiratory device 122, the user interface 124, the conduit 126, the display device 128, and the humidification tank 129 can contain one or more sensors (e.g., a pressure sensor, a flow rate sensor, or more generally any of the other sensors 130 described herein). These one or more sensors can be use, for example, to measure the air pressure and/or flow rate of pressurized air supplied by the respiratory device 122.

The display device 128 is generally used to display image(s) including still images, video images, or both and/or information regarding the respiratory device 122. For example, the display device 128 can provide information regarding the status of the respiratory device 122 (e.g., whether the respiratory device 122 is on/off, the pressure of the air being delivered by the respiratory device 122, the temperature of the air being delivered by the respiratory device 122, etc.) and/or other information (e.g., a sleep score and/or a therapy score, also referred to as a myAir^{™} score, such as described in WO 2016/061 629; the current date/time; personal information for the user 210; etc.). In some implementations, the display device 128 acts as a human-machine interface (HMI) that includes a graphic user interface (GUI) configured to display the image(s) as an input interface. The display device 128 can be an LED display, an OLED display, an LCD display, or the like. The input interface can be, for example, a touchscreen or touch-sensitive substrate, a mouse, a keyboard, or any sensor system configured to sense inputs made by a human user interacting with the respiratory device 122.

The humidification tank 129 is coupled to or integrated in the respiratory device 122 and includes a reservoir of water that can be used to humidify the pressurized air delivered from the respiratory device 122. The respiratory device 122 can include a heater to heat the water in the humidification tank 129 in order to humidify the pressurized air provided to the user. Additionally, in some implementations, the conduit 126 can also include a heating element (e.g., coupled to and/or imbedded in the conduit 126) that heats the pressurized air delivered to the user. The humidification tank 129 can be fluidly coupled to a water vapor inlet of the air pathway and deliver water vapor into the air pathway via the water vapor inlet, or can be formed in-line with the air pathway as part of the air pathway itself.

The respiratory therapy system 120 can be used, for example, as a ventilator or as a positive airway pressure (PAP) system such as a continuous positive airway pressure (CPAP) system, (an automatic positive airway pressure system (APAP), a bi-level or variable positive airway pressure system (BPAP or VPAP), or any combination thereof. The CPAP system delivers a predetermined air pressure (e.g., determined by a sleep physician) to the user. The APAP system automatically varies the air pressure delivered to the user based on, for example, respiration data associated with the user. The BPAP or VPAP system is configured to deliver a first predetermined pressure (e.g., an inspiratory positive airway pressure or IPAP) and a second predetermined pressure (e.g., an expiratory positive airway pressure or EPAP) that is lower than the first predetermined pressure.

Referring to FIG. 2, a portion of the system 100 (FIG. 1), according to some implementations, is illustrated. A user 210 of the respiratory therapy system 120 and a bed partner 220 are located in a bed 230 and are laying on a mattress 232. The user interface 124 (also referred to herein as a mask, e.g., a full face mask, a nasal mask, a nasal pillows mask, etc.) can be worn by the user 210 during a sleep session. The user interface 124 is fluidly coupled and/or connected to the respiratory device 122 via the conduit 126. In turn, the respiratory device 122 delivers pressurized air to the user 210 via the conduit 126 and the user interface 124 to increase the air pressure in the throat of the user 210 to aid in preventing the airway from closing and/or narrowing during sleep. The respiratory device 122 can be positioned on a nightstand 240 that is directly adjacent to the bed 230 as shown in FIG. 2, or more generally, on any surface or structure that is generally adjacent to the bed 230 and/or the user 210.

Referring to back to FIG. 1, the one or more sensors 130 of the system 100 include a pressure sensor 132, a flow rate sensor 134, temperature sensor 136, a motion sensor 138, a microphone 140, a speaker 142, a radio-frequency (RF) receiver 146, a RF transmitter 148, a camera 150, an infrared sensor 152, a photoplethysmogram (PPG) sensor 154, an electrocardiogram (ECG) sensor 156, an electroencephalography (EEG) sensor 158, a capacitive sensor 160, a force sensor 162, a strain gauge sensor 164, an electromyography (EMG) sensor 166, an oxygen sensor 168, an analyte sensor 174, a moisture sensor 176, a LiDAR sensor 178, or any combination thereof. Generally, each of the one or more sensors 130 are configured to output sensor data that is received and stored in the memory device 114 or one or more other memory devices.

While the one or more sensors 130 are shown and described as including each of the pressure sensor 132, the flow rate sensor 134, the temperature sensor 136, the motion sensor 138, the microphone 140, the speaker 142, the RF receiver 146, the RF transmitter 148, the camera 150, the infrared sensor 152, the photoplethysmogram (PPG) sensor 154, the electrocardiogram (ECG) sensor 156, the electroencephalography (EEG) sensor 158, the capacitive sensor 160, the force sensor 162, the strain gauge sensor 164, the electromyography (EMG) sensor 166, the oxygen sensor 168, the analyte sensor 174, the moisture sensor 176, and the LiDAR sensor 178 more generally, the one or more sensors 130 can include any combination and any number of each of the sensors described and/or shown herein.

As described herein, the system 100 generally can be used to generate physiological data associated with a user (e.g., a user of the respiratory therapy system 120 shown in FIG. 2) during a sleep session. The physiological data can be analyzed to generate one or more sleep-related parameters, which can include any parameter, measurement, etc. related to the user during the sleep session. The one or more sleep-related parameters that can be determined for the user 210 during the sleep session include, for example, an Apnea-Hypopnea Index (AHI) score, a sleep score, a flow signal, a respiration signal, a respiration rate, an inspiration amplitude, an expiration amplitude, an inspiration-expiration ratio, a number of events per hour, a pattern of events, a sleep stage and/or sleep state, pressure settings of the respiratory device 122, a heart rate, a heart rate variability, movement of the user 210, temperature, EEG activity, EMG activity, arousal, snoring, choking, coughing, whistling, wheezing, or any combination thereof. The physiological data may be additionally be generated based the data generated by the electronic circuit, or one or more components thereof, described herein.

The one or more sensors 130 can be used to generate, for example, physiological data, audio data, or both. Physiological data generated by one or more of the sensors 130 can be used by the control system 110 to determine a sleep-wake signal associated with the user 210 (FIG. 2) during the sleep session and one or more sleep-related parameters. The sleep-wake signal can be indicative of one or more sleep states, including wakefulness, relaxed wakefulness, micro-awakenings, or distinct sleep stages such as, for example, a rapid eye movement (REM) stage, a first non-REM stage (often referred to as "N1"), a second non-REM stage (often referred to as "N2"), a third non-REM stage (often referred to as "N3"), or any combination thereof. Methods for determining sleep states and/or sleep stages from physiological data generated by one or more sensors, such as the one or more sensors 130, are described in, for example, WO 2014/047310, US 2014/0088373, WO 2017/132726, WO 2019/122413, and WO 2019/122414.

In some implementations, the sleep-wake signal described herein can be timestamped to indicate a time that the user enters the bed, a time that the user exits the bed, a time that the user attempts to fall asleep, etc. The sleep-wake signal can be measured by the one or more sensors130 during the sleep session at a predetermined sampling rate, such as, for example, one sample per second, one sample per 30 seconds, one sample per minute, etc. In some implementations, the sleep-wake signal can also be indicative of a respiration signal, a respiration rate, an inspiration amplitude, an expiration amplitude, an inspiration-expiration ratio, a number of events per hour, a pattern of events, pressure settings of the respiratory device 122, or any combination thereof during the sleep session. The event(s) can include snoring, apneas, central apneas, obstructive apneas, mixed apneas, hypopneas, a mask leak (e.g., from the user interface 124), a restless leg, a sleeping disorder, choking, an increased heart rate, labored breathing, an asthma attack, an epileptic episode, a seizure, or any combination thereof. The one or more sleep-related parameters that can be determined for the user during the sleep session based on the sleep-wake signal include, for example, a total time in bed, a total sleep time, a sleep onset latency, a wake-after-sleep-onset parameter, a sleep efficiency, a fragmentation index, or any combination thereof. As described in further detail herein, the physiological data and/or the sleep-related parameters can be analyzed to determine one or more sleep-related scores.

Physiological data and/or audio data generated by the one or more sensors 130 can also be used to determine a respiration signal associated with a user during a sleep session. The respiration signal is generally indicative of respiration or breathing of the user during the sleep session. The respiration signal can be indicative of and/or analyzed to determine (e.g., using the control system 110) one or more sleep-related parameters, such as, for example, a respiration rate, a respiration rate variability, an inspiration amplitude, an expiration amplitude, an inspiration-expiration ratio, an occurrence of one or more events, a number of events per hour, a pattern of events, a sleep state, a sleet stage, an apnea-hypopnea index (AHI), pressure settings of the respiratory device 122, or any combination thereof. The one or more events can include snoring, apneas, central apneas, obstructive apneas, mixed apneas, hypopneas, a mask leak (e.g., from the user interface 124), a cough, a restless leg, a sleeping disorder, choking, an increased heart rate, labored breathing, an asthma attack, an epileptic episode, a seizure, increased blood pressure, or any combination thereof. Many of the described sleep-related parameters are physiological parameters, although some of the sleep-related parameters can be considered to be non-physiological parameters. Other types of physiological and/or non-physiological parameters can also be determined, either from the data from the one or more sensors 130, or from other types of data

As used herein, a sleep session can be defined in multiple ways. For example, a sleep session can be defined by an initial start time and an end time. In some implementations, a sleep session is a duration where the user is asleep, that is, the sleep session has a start time and an end time, and during the sleep session, the user does not wake until the end time. That is, any period of the user being awake is not included in a sleep session. From this first definition of sleep session, if the user wakes ups and falls asleep multiple times in the same night, each of the sleep intervals separated by an awake interval is a sleep session.

Alternatively, in some implementations, a sleep session has a start time and an end time, and during the sleep session, the user can wake up, without the sleep session ending, so long as a continuous duration that the user is awake is below an awake duration threshold. The awake duration threshold can be defined as a percentage of a sleep session. The awake duration threshold can be, for example, about twenty percent of the sleep session, about fifteen percent of the sleep session duration, about ten percent of the sleep session duration, about five percent of the sleep session duration, about two percent of the sleep session duration, etc., or any other threshold percentage. In some implementations, the awake duration threshold is defined as a fixed amount of time, such as, for example, about one hour, about thirty minutes, about fifteen minutes, about ten minutes, about five minutes, about two minutes, etc., or any other amount of time.

In some implementations, a sleep session is defined as the entire time between the time in the evening at which the user first entered the bed, and the time the next morning when user last left the bed. Put another way, a sleep session can be defined as a period of time that begins on a first date (e.g., Monday, January 6, 2020) at a first time (e.g., 10:00 PM), that can be referred to as the current evening, when the user first enters a bed with the intention of going to sleep (e.g., not if the user intends to first watch television or play with a smart phone before going to sleep, etc.), and ends on a second date (e.g., Tuesday, January 7, 2020) at a second time (e.g., 7:00 AM), that can be referred to as the next morning, when the user first exits the bed with the intention of not going back to sleep that next morning.

In some implementations, the user can manually define the beginning of a sleep session and/or manually terminate a sleep session. For example, the user can select (e.g., by clicking or tapping) one or more user-selectable element that is displayed on the display device172 of the external device 170 (FIG. 1) to manually initiate or terminate the sleep session.

Generally, the sleep session includes any point in time after the user 210 has laid or sat down in the bed 230 (or another area or object on which they intend to sleep), and has turned on the respiratory device 122 and donned the user interface 124. The sleep session can thus include time periods (i) when the user 210 is using the CPAP system but before the user 210 attempts to fall asleep (for example when the user 210 lays in the bed 230 reading a book); (ii) when the user 210 begins trying to fall asleep but is still awake; (iii) when the user 210 is in a light sleep (also referred to as stage 1 and stage 2 of non-rapid eye movement (NREM) sleep); (iv) when the user 210 is in a deep sleep (also referred to as slow-wave sleep, SWS, or stage 3 of NREM sleep); (v) when the user 210 is in rapid eye movement (REM) sleep; (vi) when the user 210 is periodically awake between light sleep, deep sleep, or REM sleep; or (vii) when the user 210 wakes up and does not fall back asleep.

The sleep session is generally defined as ending once the user 210 removes the user interface 124, turns off the respiratory device 122, and gets out of bed 230. In some implementations, the sleep session can include additional periods of time, or can be limited to only some of the above-disclosed time periods. For example, the sleep session can be defined to encompass a period of time beginning when the respiratory device 122 begins supplying the pressurized air to the airway or the user 210, ending when the respiratory device 122 stops supplying the pressurized air to the airway of the user 210, and including some or all of the time points in between, when the user 210 is asleep or awake.

The pressure sensor 132 outputs pressure data that can be stored in the memory device 114 and/or analyzed by the processor 112 of the control system 110. In some implementations, the pressure sensor 132 is an air pressure sensor (e.g., barometric pressure sensor) that generates sensor data indicative of the respiration (e.g., inhaling and/or exhaling) of the user of the respiratory therapy system 120 and/or ambient pressure. In such implementations, the pressure sensor 132 can be coupled to or integrated in the respiratory device 122. The pressure sensor 132 can be, for example, a capacitive sensor, an electromagnetic sensor, a piezoelectric sensor, a strain-gauge sensor, an optical sensor, a potentiometric sensor, or any combination thereof.

The flow rate sensor 134 outputs flow rate data that can be stored in the memory device 114 and/or analyzed by the processor 112 of the control system 110. Examples of flow rate sensors (such as, for example, the flow rate sensor 134) are described in WO 2012/012835. In some implementations, the flow rate sensor 134 is used to determine an air flow rate from the respiratory device 122, an air flow rate through the conduit 126, an air flow rate through the user interface 124, or any combination thereof. In such implementations, the flow rate sensor 134 can be coupled to or integrated in the respiratory device 122, the user interface 124, or the conduit 126. The flow rate sensor 134 can be a mass flow rate sensor such as, for example, a rotary flow meter (e.g., Hall effect flow meters), a turbine flow meter, an orifice flow meter, an ultrasonic flow meter, a hot wire sensor, a vortex sensor, a membrane sensor, or any combination thereof. In some implementations, the flow rate sensor 134 is configured to measure a vent flow (e.g., intentional "leak"), an unintentional leak (e.g., mouth leak and/or mask leak), a patient flow (e.g., air into and/or out of lungs), or any combination thereof. In some implementations, the flow rate data can be analyzed to determine cardiogenic oscillations of the user. In one example, the pressure sensor 132 can be used to determine a blood pressure of a user.

The temperature sensor 136 outputs temperature data that can be stored in the memory device 114 and/or analyzed by the processor 112 of the control system 110. In some implementations, the temperature sensor 136 generates temperatures data indicative of a core body temperature of the user 210 (FIG. 2), a skin temperature of the user 210, a temperature of the air flowing from the respiratory device 122 and/or through the conduit 126, a temperature in the user interface 124, an ambient temperature, or any combination thereof. The temperature sensor 136 can be, for example, a thermocouple sensor, a thermistor sensor, a silicon band gap temperature sensor or semiconductor-based sensor, a resistance temperature detector, or any combination thereof.

The microphone 140 outputs sound data that can be stored in the memory device 114 and/or analyzed by the processor 112 of the control system 110. The audio data generated by the microphone 140 is reproducible as one or more sound(s) during a sleep session (e.g., sounds from the user 210). The audio data form the microphone 140 can also be used to identify (e.g., using the control system 110) an event experienced by the user during the sleep session, as described in further detail herein. The microphone 140 can be coupled to or integrated in the respiratory device 122, the user interface 124, the conduit 126, or the external device 170. In some implementations, the system 100 includes a plurality of microphones (e.g., two or more microphones and/or an array of microphones with beamforming) such that sound data generated by each of the plurality of microphones can be used to discriminate the sound data generated by another of the plurality of microphones.

The speaker 142 outputs sound waves that are audible to a user of the system 100 (e.g., the user 210 of FIG. 2). The speaker 142 can be used, for example, as an alarm clock or to play an alert or message to the user 210 (e.g., in response to an event). In some implementations, the speaker 142 can be used to communicate the audio data generated by the microphone 140 to the user. The speaker 142 can be coupled to or integrated in the respiratory device 122, the user interface 124, the conduit 126, or the external device 170.

The microphone 140 and the speaker 142 can be used as separate devices. In some implementations, the microphone 140 and the speaker 142 can be combined into an acoustic sensor 141 (e.g., a SONAR sensor), as described in, for example, WO 2018/050913 and WO 2020/104465. In such implementations, the speaker 142 generates or emits sound waves at a predetermined interval and the microphone 140 detects the reflections of the emitted sound waves from the speaker 142. The sound waves generated or emitted by the speaker 142 have a frequency that is not audible to the human ear (e.g., below 20 Hz or above around 18 kHz) so as not to disturb the sleep of the user 210 or the bed partner 220 (FIG. 2). Based at least in part on the data from the microphone 140 and/or the speaker 142, the control system 110 can determine a location of the user 210 (FIG. 2) and/or one or more of the sleep-related parameters described in herein, such as, for example, a respiration signal, a respiration rate, an inspiration amplitude, an expiration amplitude, an inspiration-expiration ratio, a number of events per hour, a pattern of events, a sleep state, a sleep stage, pressure settings of the respiratory device 122, or any combination thereof. In such a context, a sonar sensor may be understood to concern an active acoustic sensing, such as by generating and/or transmitting ultrasound and/or low frequency ultrasound sensing signals (e.g., in a frequency range of about 17-23 kHz, 18-22 kHz, or 17-18 kHz, for example), through the air. Such a system may be considered in relation to WO 2018/050913 and WO 2020/104465 mentioned above.

In some implementations, the sensors 130 include (i) a first microphone that is the same as, or similar to, the microphone 140, and is integrated in the acoustic sensor 141 and (ii) a second microphone that is the same as, or similar to, the microphone 140, but is separate and distinct from the first microphone that is integrated in the acoustic sensor 141.

The RF transmitter 148 generates and/or emits radio waves having a predetermined frequency and/or a predetermined amplitude (e.g., within a high frequency band, within a low frequency band, long wave signals, short wave signals, etc.). The RF receiver 146 detects the reflections of the radio waves emitted from the RF transmitter 148, and this data can be analyzed by the control system 110 to determine a location of the user 210 (FIG. 2) and/or one or more of the sleep-related parameters described herein. An RF receiver (either the RF receiver 146 and the RF transmitter 148 or another RF pair) can also be used for wireless communication between the control system 110, the respiratory device 122, the one or more sensors 130, the external device 170, or any combination thereof. While the RF receiver 146 and RF transmitter 148 are shown as being separate and distinct elements in FIG. 1, in some implementations, the RF receiver 146 and RF transmitter 148 are combined as a part of an RF sensor 147 (e.g., a RADAR sensor).. In some such implementations, the RF sensor 147 includes a control circuit. The specific format of the RF communication can be Wi-Fi, Bluetooth, or the like.

In some implementations, the RF sensor 147 is a part of a mesh system. One example of a mesh system is a Wi-Fi mesh system, which can include mesh nodes, mesh router(s), and mesh gateway(s), each of which can be mobile/movable or fixed. In such implementations, the Wi-Fi mesh system includes a Wi-Fi router and/or a Wi-Fi controller and one or more satellites (e.g., access points), each of which include an RF sensor that the is the same as, or similar to, the RF sensor 147. The Wi-Fi router and satellites continuously communicate with one another using Wi-Fi signals. The Wi-Fi mesh system can be used to generate motion data based on changes in the Wi-Fi signals (e.g., differences in received signal strength) between the router and the satellite(s) due to an object or person moving partially obstructing the signals. The motion data can be indicative of motion, breathing, heart rate, gait, falls, behavior, etc., or any combination thereof.

The camera 150 outputs image data reproducible as one or more images (e.g., still images, video images, thermal images, or any combination thereof) that can be stored in the memory device 114. The image data from the camera 150 can be used by the control system 110 to determine one or more of the sleep-related parameters described herein, such as, for example, one or more events (e.g., periodic limb movement or restless leg syndrome), a respiration signal, a respiration rate, an inspiration amplitude, an expiration amplitude, an inspiration-expiration ratio, a number of events per hour, a pattern of events, a sleep state, a sleep stage, or any combination thereof. Further, the image data from the camera 150 can be used to, for example, identify a location of the user, to determine chest movement of the user 210 (FIG. 2), to determine air flow of the mouth and/or nose of the user 210, to determine a time when the user 210 enters the bed 230 (FIG. 2), and to determine a time when the user 210 exits the bed 230. In some implementations, the camera 150 includes a wide angle lens or a fisheye lens.

The infrared (IR) sensor 152 outputs infrared image data reproducible as one or more infrared images (e.g., still images, video images, or both) that can be stored in the memory device 114. The infrared data from the IR sensor 152 can be used to determine one or more sleep-related parameters during a sleep session, including a temperature of the user 210 and/or movement of the user 210. The IR sensor 152 can also be used in conjunction with the camera 150 when measuring the presence, location, and/or movement of the user 210. The IR sensor 152 can detect infrared light having a wavelength between about 700 nm and about 1 mm, for example, while the camera 150 can detect visible light having a wavelength between about 380 nm and about 740 nm.

The PPG sensor 154 outputs physiological data associated with the user 210 (FIG. 2) that can be used to determine one or more sleep-related parameters, such as, for example, a heart rate, a heart rate variability, a cardiac cycle, respiration rate, an inspiration amplitude, an expiration amplitude, an inspiration-expiration ratio, estimated blood pressure parameter(s), or any combination thereof. The PPG sensor 154 can be worn by the user 210, embedded in clothing and/or fabric that is worn by the user 210, embedded in and/or coupled to the user interface 124 and/or its associated headgear (e.g., straps, etc.), etc.

The ECG sensor 156 outputs physiological data associated with electrical activity of the heart of the user 210. In some implementations, the ECG sensor 156 includes one or more electrodes that are positioned on or around a portion of the user 210 during the sleep session. The physiological data from the ECG sensor 156 can be used, for example, to determine one or more of the sleep-related parameters described herein.

The EEG sensor 158 outputs physiological data associated with electrical activity of the brain of the user 210. In some implementations, the EEG sensor 158 includes one or more electrodes that are positioned on or around the scalp of the user 210 during the sleep session. The physiological data from the EEG sensor 158 can be used, for example, to determine a sleep state and/or a sleep stage of the user 210 at any given time during the sleep session. In some implementations, the EEG sensor 158 can be integrated in the user interface 124 and/or the associated headgear (e.g., straps, etc.).

The capacitive sensor 160, the force sensor 162, and the strain gauge sensor 164 output data that can be stored in the memory device 114 and used by the control system 110 to determine one or more of the sleep-related parameters described herein. The EMG sensor 166 outputs physiological data associated with electrical activity produced by one or more muscles. The oxygen sensor 168 outputs oxygen data indicative of an oxygen concentration of gas (e.g., in the conduit 126 or at the user interface 124). The oxygen sensor 168 can be, for example, an ultrasonic oxygen sensor, an electrical oxygen sensor, a chemical oxygen sensor, an optical oxygen sensor, a pulse oximeter (e.g., SpO₂ sensor), or any combination thereof. In some implementations, the one or more sensors 130 also include a galvanic skin response (GSR) sensor, a blood flow sensor, a respiration sensor, a pulse sensor, a sphygmomanometer sensor, an oximetry sensor, or any combination thereof.

The analyte sensor 174 can be used to detect the presence of an analyte in the exhaled breath of the user 210. The data output by the analyte sensor 174 can be stored in the memory device 114 and used by the control system 110 to determine the identity and concentration of any analytes in the breath of the user 210. In some implementations, the analyte sensor 174 is positioned near a mouth of the user 210 to detect analytes in breath exhaled from the user 210's mouth. For example, when the user interface 124 is a facial mask that covers the nose and mouth of the user 210, the analyte sensor 174 can be positioned within the facial mask to monitor the user 210's mouth breathing. In other implementations, such as when the user interface 124 is a nasal mask or a nasal pillow mask, the analyte sensor 174 can be positioned near the nose of the user 210 to detect analytes in breath exhaled through the user's nose. In still other implementations, the analyte sensor 174 can be positioned near the user 210's mouth when the user interface 124 is a nasal mask or a nasal pillow mask. In this implementation, the analyte sensor 174 can be used to detect whether any air is inadvertently leaking from the user 210's mouth. In some implementations, the analyte sensor 174 is a volatile organic compound (VOC) sensor that can be used to detect carbon-based chemicals or compounds. In some implementations, the analyte sensor 174 can also be used to detect whether the user 210 is breathing through their nose or mouth. For example, if the data output by an analyte sensor 174 positioned near the mouth of the user 210 or within the facial mask (in implementations where the user interface 124 is a facial mask) detects the presence of an analyte, the control system 110 can use this data as an indication that the user 210 is breathing through their mouth.

The moisture sensor 176 outputs data that can be stored in the memory device 114 and used by the control system 110. The moisture sensor 176 can be used to detect moisture in various areas surrounding the user (e.g., inside the conduit 126 or the user interface 124, near the user 210's face, near the connection between the conduit 126 and the user interface 124, near the connection between the conduit 126 and the respiratory device 122, etc.). Thus, in some implementations, the moisture sensor 176 can be coupled to or integrated in the user interface 124 or in the conduit 126 to monitor the humidity of the pressurized air from the respiratory device 122. In other implementations, the moisture sensor 176 is placed near any area where moisture levels need to be monitored. The moisture sensor 176 can also be used to monitor the humidity of the ambient environment surrounding the user 210, for example, the air inside the bedroom.

The Light Detection and Ranging (LiDAR) sensor 178 can be used for depth sensing. This type of optical sensor (e.g., laser sensor) can be used to detect objects and build three dimensional (3D) maps of the surroundings, such as of a living space. LiDAR can generally utilize a pulsed laser to make time of flight measurements. LiDAR is also referred to as 3D laser scanning. In an example of use of such a sensor, a fixed or mobile device (such as a smartphone) having a LiDAR sensor 166 can measure and map an area extending 5 meters or more away from the sensor. The LiDAR data can be fused with point cloud data estimated by an electromagnetic RADAR sensor, for example. The LiDAR sensor(s) 178 can also use artificial intelligence (AI) to automatically geofence RADAR systems by detecting and classifying features in a space that might cause issues for RADAR systems, such a glass windows (which can be highly reflective to RADAR). LiDAR can also be used to provide an estimate of the height of a person, as well as changes in height when the person sits down, or falls down, for example. LiDAR may be used to form a 3D mesh representation of an environment. In a further use, for solid surfaces through which radio waves pass (e.g., radio-translucent materials), the LiDAR may reflect off such surfaces, thus allowing a classification of different type of obstacles.

In some implementations, the one or more sensors 130 also include a galvanic skin response (GSR) sensor, a blood flow sensor, a respiration sensor, a pulse sensor, a sphygmomanometer sensor, an oximetry sensor, a sonar sensor, a RADAR sensor, a blood glucose sensor, a color sensor, a pH sensor, an air quality sensor, a tilt sensor, a rain sensor, a soil moisture sensor, a water flow sensor, an alcohol sensor, or any combination thereof.

While shown separately in FIG. 1, any combination of the one or more sensors 130 can be integrated in and/or coupled to any one or more of the components of the system 100, including the respiratory device 122, the user interface 124, the conduit 126, the humidification tank 129, the control system 110, the external device 170, or any combination thereof. For example, the microphone 140 and the speaker 142 can be integrated in and/or coupled to the external device 170; and the pressure sensor 132 and/or flow rate sensor 134 are integrated in and/or coupled to the respiratory device 122. In some implementations, at least one of the one or more sensors 130 is not coupled to the respiratory device 122, the control system 110, or the external device 170, and is positioned generally adjacent to the user 210 during the sleep session (e.g., positioned on or in contact with a portion of the user 210, worn by the user 210, coupled to or positioned on the nightstand, coupled to the mattress, coupled to the ceiling, etc.).

The data from the one or more sensors 130 can be analyzed to determine one or more sleep-related parameters, which can include a respiration signal, a respiration rate, a respiration pattern, an inspiration amplitude, an expiration amplitude, an inspiration-expiration ratio, an occurrence of one or more events, a number of events per hour, a pattern of events, a sleep state, an apnea-hypopnea index (AHI), or any combination thereof. The one or more events can include snoring, apneas, central apneas, obstructive apneas, mixed apneas, hypopneas, a mask leak, a cough, a restless leg, a sleeping disorder, choking, an increased heart rate, labored breathing, an asthma attack, an epileptic episode, a seizure, increased blood pressure, or any combination thereof. Many of these sleep-related parameters are physiological parameters, although some of the sleep-related parameters can be considered to be non-physiological parameters. Other types of physiological and non-physiological parameters can also be determined, either from the data from the one or more sensors 130, and/or from other types of data such as the data generated by the electronic circuit, or one or more components thereof, described herein.

The external device 170 (FIG. 1) includes a display device 172. The external device 170 can be, for example, a mobile device such as a smart phone, a tablet, a gaming console, a smart watch, a laptop, or the like. Alternatively, the external device 170 can be an external sensing system, a television (e.g., a smart television) or another smart home device (e.g., a smart speaker(s) such as Google Home, Amazon Echo, Alexa etc.). In some implementations, the external device is a wearable device (e.g., a smart watch). The display device 172 is generally used to display image(s) including still images, video images, or both. In some implementations, the display device 172 acts as a human-machine interface (HMI) that includes a graphic user interface (GUI) configured to display the image(s) and an input interface. The display device 172 can be an LED display, an OLED display, an LCD display, or the like. The input interface can be, for example, a touchscreen or touch-sensitive substrate, a mouse, a keyboard, or any sensor system configured to sense inputs made by a human user interacting with the external device 170. In some implementations, one or more external devices can be used by and/or included in the system 100.

While the control system 110 and the memory device 114 are described and shown in FIG. 1 as being a separate and distinct component of the system 100, in some implementations, the control system 110 and/or the memory device 114 are integrated in the external device 170 and/or the respiratory device 122. Alternatively, in some implementations, the control system 110 or a portion thereof (e.g., the processor 112) can be located in a cloud (e.g., integrated in a server, integrated in an Internet of Things (IoT) device, connected to the cloud, be subject to edge cloud processing, etc.), located in one or more servers (e.g., remote servers, local servers, etc., or any combination thereof.

In some implementations, the system 100 further includes an electronic circuit 180. The electronic circuit 180 can include one or more conductors 182, one or more electronic components 184, one or more current sensors 186, or any combination thereof. In some implementations, at least a portion of the electronic circuit 180 is coupled to and/or integrated in the conduit 126 of the respiratory therapy system 120. In some implementations, at least a portion of the electronic circuit 180 is coupled to and/or integrated in the respiratory device 122 of the respiratory therapy system 120. In some implementations, at least a portion of the electronic circuit 180 is coupled to and/or integrated in the user interface 124 of the respiratory therapy system 120. In some implementations, at least a portion of the electronic circuit 180 is coupled to and/or integrated in the display device 128 of the respiratory therapy system 120. In some implementations, at least a portion of the electronic circuit 180 is coupled to and/or integrated in the humidification tank 129 of the respiratory therapy system 120. Additionally, in some implementations, one or more electronic components 184 of the electronic circuit 180 is coupled to a conductor of the one or more conductors 182. A current sensor of the one or more current sensors 186 can also be coupled to the conductor, and is configured to (i) measure a current flowing through the electronic circuit 180, and (ii) generate data associated with the conductor and the electronic components coupled to the conductor.

While system 100 is shown as including all of the components described above, more or fewer components can be included in a system according to implementations of the present disclosure. For example, a first alternative system includes the control system 110, the memory device 114, and at least one of the one or more sensors 130. As another example, a second alternative system includes the control system 110, the memory device 114, at least one of the one or more sensors 130, and the external device 170. As yet another example, a third alternative system includes the control system 110, the memory device 114, the respiratory therapy system 120, at least one of the one or more sensors 130, and the external device 170. Thus, various systems can be formed using any portion or portions of the components shown and described herein and/or in combination with one or more other components.

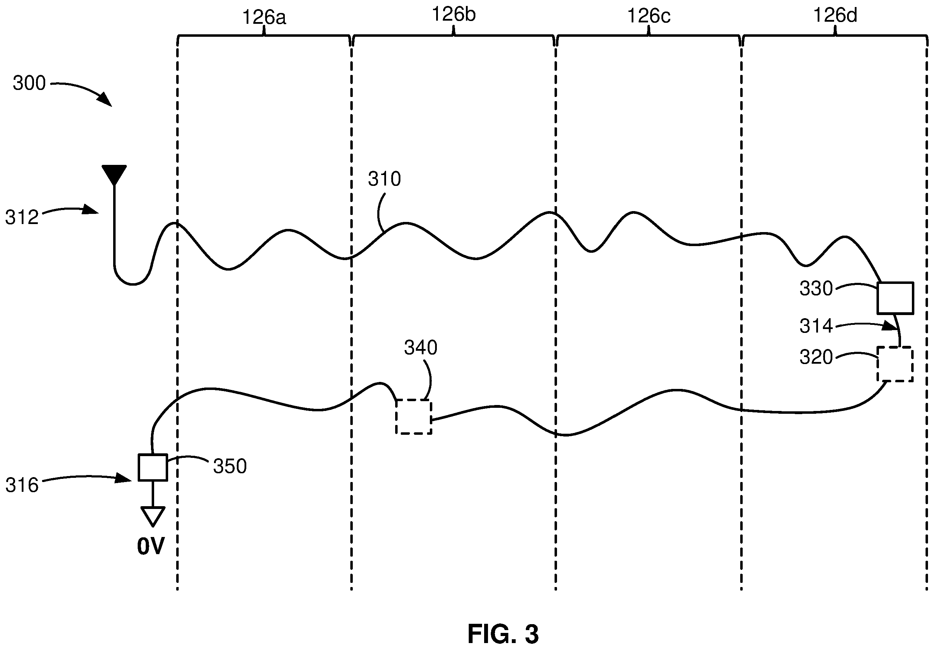

Referring to FIG. 3, an electronic circuit 300, which is the same as, or similar to, the electronic circuit 180 of the system 100 (FIG. 1) is depicted, according to some implementations of the present disclosure. The electronic circuit 300 can include a conductor (e.g., the wire 310), a first electronic component 330, and a current sensor 350, which are the same as, or similar to, the one or more conductors 182, the one or more electronic components 184, and the one or more current sensors 186 of the system 100 as shown in FIG. 1. The electronic circuit 300 is coupled to a conduit, such as the conduit 126 of the respiratory therapy system 120 (FIGS. 1-2). For example, as shown in FIG. 2, the conduit 126 allows the flow of air between two components of the respiratory therapy system 120, such as the respiratory device 122 and the user interface 124. The user interface 124 is fluidly coupled and/or connected to the respiratory device 122 via the conduit 126. In turn, the respiratory device 122 delivers pressurized air to the user 210 via the conduit 126 and the user interface 124 to, in certain implementations, increase the air pressure in the throat of the user 210 to aid in preventing the airway from closing and/or narrowing during sleep. In some implementations, the conduit is not limited to the conduit 126 of the respiratory therapy system 120. For example, the conduit can include a ventilator conduit, or the like.

The conduit 126 can include a plurality of segments, such as segments 126a, 126b, 126c, and 126d. The first segment 126a is positioned near the respiratory device 122. The last segment 126d is positioned near the user interface 124, and therefore near the user 210. In some implementations, at least a segment of the electronic circuit 300 (FIG. 3) is physically integrated into the conduit 126. For example, in some implementations, a first segment of the electronic circuit 300 is physically integrated into the conduit 126, and a second segment of the electronic circuit 300 is physically positioned within the respiratory device 122 that is coupled to the conduit 126. As such, in some implementations, movement of the first segment of the electronic circuit 300 is indicative of a corresponding movement of at least a segment of the conduit 126.

The electronic circuit 300 includes the conductor (e.g., the wire 310) that is coupled to a power source at a first end 312 of the wire 310. The wire 310 can be made of any suitable material, such as metal. For example, the wire 310 can be made of beryllium, copper, or both. Therefore, in some implementations, movement in a segment of the wire 310 is indicative of a corresponding movement of a segment of the conduit 126. For example, as best shown in FIG. 3, movement in a segment of the wire 310 near the point 314 of the wire 310 is indicative of a corresponding movement of the segment 126d of the conduit 126. As another example, movement in a segment of the wire 310 near the point 316 of the wire 310 is indicative of a corresponding movement of the segment 126a of the conduit 126.

The electronic circuit 300 includes a first electronic component 330 (e.g., the same as, or similar to, the one or more electronic components 184 of the system 100) coupled to the wire 310. In some implementations, the first electronic component 330 has first electrical property that is configured to change (e.g., from a first value to a second value) based at least in part on movement of the first electronic component 330. The movement of the first electronic component 330 is indicative of movement of a corresponding portion of the wire 310, which in turn is indicative of movement of a corresponding portion (e.g., the segment 126d) of the conduit 126 (FIGS. 1-2).

In some implementations, the movement data can be used to determine sleep-related activities (e.g., bruxism, sleep state, sleep stage, body position, head position, etc.) associated with the user. Therefore, in some such implementations, the first electronic component 330 is positioned adjacent to the user interface 124, which is directly couplable to the user's face. Placement of the first electronic component 330 towards the user is advantageous because (i) it can detect movement and/or position of the user's head, and (ii) it can eliminate non-user movement (e.g., someone else accidentally moved the conduit 126). That is, the first electronic component 330 can advantageously be located at, or proximal to, the portion of the wire 310 or conduit 126 nearest the user/user interface 124, which is typically distal to the first end 312 of the wire 310 or respiratory device 114.

In some implementations, the information (e.g., the movement data) associated with when the conduit and/or the user is moving and/or has moved during therapy and/or sleep session includes real-time information. In some implementations, the same information might also be useful after that therapy and/or sleep session.

It is contemplated that the first electronic component 330 can include one or more resistors, one or more semiconductors (e.g., transistors, diodes, etc.), one or more capacitors, one or more inductors, one or more ferroelectric sensors (e.g., PVDF sensors), one or more piezoelectric sensors, one or more graphene sensors, one or more optical sensors (e.g., an optical system comprising an optical source (e.g. optical transmitter), an optical transmission line (e.g., optical fiber), and/or an optical receiver), one or more gyroscopes, one or more accelerometers, or any combination thereof.

For example, the first electronic component 330 can include a resistor, which has a resistance configured to change based at least in part on movement of the resistor. A standard resistance of the resistor is between about 100 ohms to about 100,000 ohms, such as about 10,000 ohms. The standard resistance can be measured when the resistor is at rest (e.g., when the resistor is not moving, not bent, not twisted, not stretched, not compressed, etc.).

As another example, the first electronic component 330 can include a capacitor, which has a capacitance configured to change based at least in part on movement of the capacitor. A standard capacitance of the capacitor is about 1 picofarad (pF) to 100 nanofarads (nF).

As a further example, the first electronic component 330 can include an inductor, which has an inductance configured to change based at least in part on movement of the inductor. A standard inductance of the inductor is about 1 nanohenry (nH) to 100 micohenrys (mH). In some implementations, changes in wire inductance variation due to conduit movements can be measured, which may be achieved by using the existing conduit (e.g., tube) "as is" and using a heater circuit current inductance sensor. In some implementations, modifications could be made to the conduit (e.g., the tube) to improve the induction variation with movement.

Both capacitance (e.g., the ability to store electric charge energy) and inductance (e.g., the ability to store moving charge energy) are determined by the dimensions and/or topology of the component. A change in structural dimensions (due to, e.g., deformation of the physical structure of the component) results in a change in the respective parameter. For example, the inductance of a solenoid similar to the coiled heater component in the CPAP heated tube is given by L = uN² A/L, where u is the permittivity of the tube volume (=1 for air), N is the number of turns of wire, A is the cross sectional area, and L is the tube length. With u and N relatively fixed, any change in the area A or length L will result in an inductance change. Therefore, in some implementations, to design the component, the values of N, A, and L can be optimized to provide maximum sensitivity to movement.

In some implementations, the electronic circuit 300 includes a second electronic component 320 (e.g., the same as, or similar to, the one or more electronic components 184 of the system 100) coupled to the wire 310. The second electronic component 320 has a second electrical property that is configured to change based at least in part on a temperature of the second electronic component 320. For example, in some implementations, the second electronic component 320 is a main resistor (e.g., a "thermistor") that may be positioned about midway (at point 314) on the wire 310. The resistance of the main resistor 320 is configured to change based at least in part on the temperature of the main resistor 320. The main resistor 320 includes a second resistance that is greater than the first resistance of first electronic component 330 (e.g., the "secondary" resistor). As such, the main resistor 320 changes at a greater degree with temperature, than does the secondary resistor 330 with movement. In some implementations, the system 100 may be configured to perform temperature calibration based at least in part on the current temperatures of the main resister 320 and/or the secondary resistor 330 of the electronic circuit 300.

For example, in some implementations, the second resistance of the main resistor 320 is about 2 times, 3 times, 5 times, 10 times, 15 times, 20 times, 30 times, 40 times, or 50 times of the secondary resistor 330. Additionally, or alternatively, in some implementations, the first resistance of the secondary resistor 330 is between about 5 ohms to about 5,000 ohms, such as about 1,000 ohms. In some implementations, the second resistance of the main resistor 320 is between about 5,000 ohms to about 50,000 ohms, such as about 10,000 ohms.

Additionally or alternatively, in some implementations, the electronic circuit 300 may include a motion sensor (e.g., the motion sensor 138 of the system 100), such as a gyroscope and/or an accelerometer. The motion sensor 138 may be in place of, or in addition to, the first electronic component 330 and/or the second electronic component 320. In some such implementations, while the motion sensor 138 can detect movement with respect to the segment of the conduit where the motion sensor 138 is located, it may not be able to detect the temperature, the angle, movement of other segments of the conduit, and/or how stretched the conduit is. Thus, having the first electronic component 330 and/or the second electronic component 320 being different from the motion sensor 138 may be advantageous in providing a holistic determination of the conduit movement.

In some implementations, electronic circuit 300 includes the current sensor 350. The current sensor 350 can be positioned anywhere on the wire, such as at the second end 316 of the wire 310. In some implementations, at least a portion of the electronic circuit 300 can be positioned within the conduit 126, while the current sensor 350 is positioned outside the conduit 126. For example, in some such implementations, the current sensor 350 can be positioned within the respiratory device 122 (FIGS. 1-2), extending out from the first segment 126a. The current sensor 350 is configured to (i) measure a current flowing through the electronic circuit 300, and (ii) generate the received data associated with the first electrical property (e.g., resistance) of the secondary resistor 330. In some implementations, the received data is current data that is associated with the measured current flowing through the electronic circuit 300. Thus, the analysis of the received data can include identifying changes in the current flowing through the electronic circuit 300, which is also indicative of the changes in resistance in the electronic circuit 300, which changes may be caused by movements of the first electronic component 330 (e.g., the secondary resistor).

In some implementations, the analysis of the received data further includes identifying a first magnitude of the identified changes in the current flowing through the electronic circuit 300. For example, the identified first magnitude can be associated with the movement of the secondary resistor 330 of the electronic circuit 300. Further, the analysis of the received data can also include identifying a second magnitude of the identified changes in the current flowing through the electronic circuit 300. For example, the identified second magnitude can be associated with the temperature of the main resistor 320 of the electronic circuit 300. In some such examples, the system 100 may be configured to perform temperature calibration based at least in part on the current temperature of the main resister 320 of the electronic circuit 300.

As discussed herein, in some implementations, the second resistance of the main resistor 320 is greater than the first resistance of secondary resistor 330. As such, in some implementations, the identified second magnitude (e.g., associated with the temperature of the main resistor 320) is less than the identified first magnitude (e.g., associated with the movement of the secondary resistor 330).

Additionally, or alternatively, the analysis of the received data includes identifying a first rate of change of a magnitude of the current flowing through the electronic circuit 300. For example, the identified first rate of change can be associated with the movement of the secondary resistor 330 of the electronic circuit 300. Further, the analysis of the received data can also include identifying a second rate of change of the magnitude of the identified changes in the current flowing through the electronic circuit 300. For example, the identified second rate of change can be associated with the temperature of the main resistor 320 of the electronic circuit 300. As such, changes associated with the temperature of the main resistor 320 can be differentiated from changes associated with the movement of the secondary resistor 330, because the identified second rate of change is different from the identified first rate of change.

In some implementations, the rate of change can also be referred to as frequency. In some such implementations, because changes in movement typically occur much faster than changes in temperature, a higher frequency (e.g., a faster rate of change) can be indicative of the movement of the secondary resistor 330, whereas a lower frequency (e.g., a slower rate of change) can be indicative of the temperature of the main resistor 320.

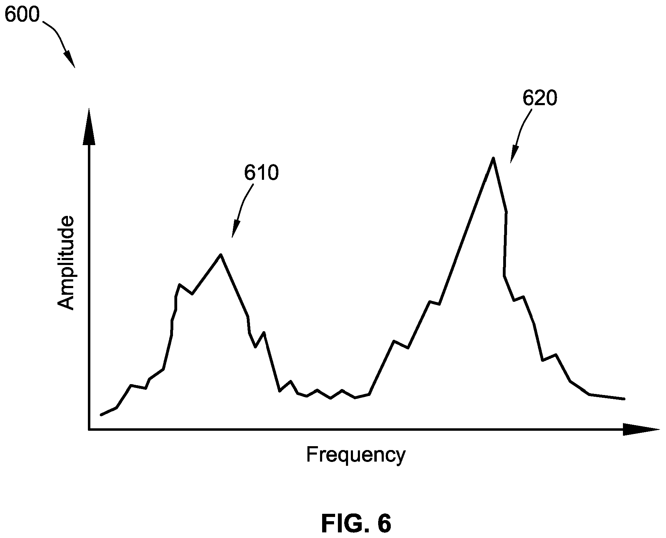

In some implementations, FFT methods can be used to separate movement data from temperature data. For example, an FFT can be used to transform the time series signal into the frequency domain. This will separate signal variations due to movements from signal variations due to temperature changes. Referring to FIG. 6, example movement data and temperature data are depicted on a frequency-amplitude plot 600, according to some implementations of the present disclosure. Typically, in the frequency domain, movement variations 620 will be represented by higher frequencies and higher amplitudes, whereas temperature variations 610 will be represented by lower frequencies and lower amplitudes. In some implementations, by summing the amplitudes of a range of higher frequencies and comparing this sum to a threshold, movement can be determined.

In some implementations, the sampling speed is adjusted accordingly (e.g., faster) for detecting and/or determining movement associated with the electronic circuit 300. For example, a system for detecting and/or determining movement of an electronic circuit can sample 64 or 128 times per second (e.g., as the faster rate). This faster "sampling" can allow filtering (e.g., low pass, high pass, band pass), which can effectively separate the current data associated with temperature changes from movement changes. For example, current data associated with movement remains after applying high pass filtering.

In some implementations, the current sensor 350 includes a voltage regulator, so that the current sensor 350 can work with any starting voltage (e.g., any point of the wire 310). As such, so long as the current sensor 350 is positioned in series of the power supply, the current sensor 350 can be located anywhere on the wire 310. For example, as shown in FIG. 3, the current sensor 350 is positioned between two points on the wire 310. The voltage difference between the two points can therefore power the current sensor 350.

In some implementations, the electronic circuit 300 includes an additional electronic component 340 (e.g., the same as, or similar to, the one or more electronic components 184 of the system 100) coupled to the wire 310 at a distance from the first electronic component 330. The additional electronic component 340 has an additional electrical property that is configured to change based at least in part on movement of the additional electronic component 340. The movement of the additional electronic component 340 is indicative of movement of a corresponding portion of the wire 310, which in turn is indicative of movement of a corresponding segment 126b of the conduit 126 (FIGS. 1-2). For example, responsive to a determination that the additional electrical property of the additional electronic component 340 has changed, it can be determined that the conduit 126 (more specifically, the segment 126b of the conduit) is moving adjacent to the additional electronic component 340.

For example, in some implementations, the additional electronic component 340 is the same as the first electronic component 330. In other words, the additional electronic component 340 and the first electronic component 330 can both be resistors, semiconductors, capacitors, inductors, ferroelectric sensors, piezoelectric sensors, graphene sensors, optical sensors, gyroscopes, accelerometers, etc. Alternatively, the additional electronic component 340 can be different from the first electronic component 330. For example, the first electronic component 330 is a resistor, and the additional electronic component 340 is an inductor. Other compatible combinations can be used, also.

In some implementations, determining whether an electrical property of an electronic component (e.g., the first electronic component 330, the second electronic component 320, and/or the additional electronic component 340) includes determining (i) a degree of change of the electrical property, (ii) a number of changes of the electrical property, (iii) a frequency of changes of the electric property, or (iv) any combination thereof. In some implementations, two or more electronic components (e.g., the first electronic component 330, the second electronic component 320, and/or the additional electronic component 340) may be positioned at about the same location and/or segment of the electronic circuit 330, where each electronic component has different sensitivities to movement. For example, each of the two or more electronic components may detect and/or record movement when the degree of movement of the conduit exceeds different thresholds. As such, in some implementations, the combination of the two or more electronic components is configured to indicate the degree of movement. In some such implementations, sensors with different sensitivities to movement may be used. For example, a micro electro mechanical system (MEMS) accelerometer would sense the acceleration of the conduit (e.g., tube) and its relative alignment with regard to gravity, while an inductive sensor would sense only the change in deformation of the conduit itself independent of gravitational and/or movement acceleration. Additionally or alternatively, in some implementations, two or more electronic components (e.g., the first electronic component 330, the second electronic component 320, and/or the additional electronic component 340) may be positioned at different locations on the electronic circuit 330, corresponding to different locations along the conduit. As such, the combination of the two or more electronic components is configured to indicate the location of movement.

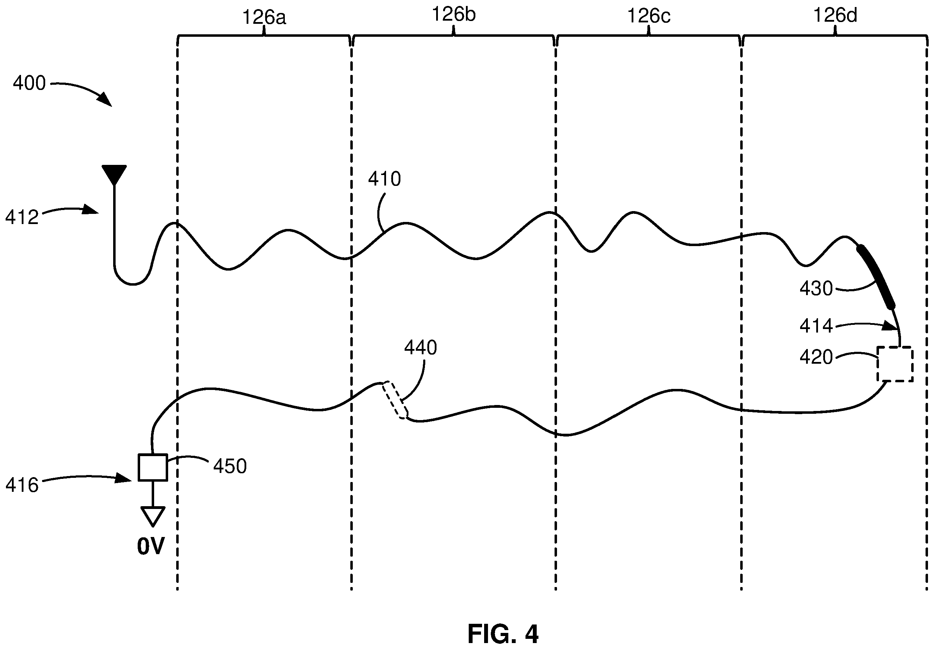

Referring now to FIG. 4, an electronic circuit 400 is depicted, according to some implementations of the present disclosure. As shown, the electronic circuit 400 is the same as, or similar to, the electronic circuit 300 in FIG. 3, where like reference numbers are used for like elements, except that the electronic circuit 400 does not include a first electronic component (e.g., the first electronic component 330 of the electronic circuit 300). Instead, a portion 430 of the electronic circuit 400 has the first electrical property that is configured to change based at least in part on movement of the portion 430 of the electronic circuit 300. In other words, portion 430 acts in an analogous manner to electronic component 330 described herein. Thus, responsive to the determination that the first electrical property of the portion 430 of the electronic circuit 400 has changed, it is determined that the conduit is moving or has moved.

In some implementations, the portion 430 of the electronic circuit 400 is a portion of the wire 410. In some such implementations, the portion 430 of the wire 410 has a resistance that is configured to change when moved. For example, when the portion 430 of the wire 410 is moved, the resistance is configured to change at least 1%, at least 2%, at least 3 %, at least 4%, at least 5%, at least 6%, at least 7%, at least 8%, at least 9%, at least 10%, at least 11%, at least 12%, at least 13%, at least 14%, or at least 15%. In some implementations, the portion 430 of the wire 410 has a resistance that is configured to change when stretched, compressed, bent, twisted, or any combination thereof. Alternatively, the entire wire 410 itself has a resistance that is configured to change when moved.

In some implementations, the first electrical property of the electronic circuit 400 is changed responsive to a change in shape, stretch, bend, or twist of the portion 430 of the wire 410, where the stretch, the bend, or the twist of the portion 430 of the wire 410 is configured to change based at least in part on the movement of the portion 430 of the wire 410.

The electronic circuit 400 in FIG. 4 can include an electronic component 420, similar to the thermistor 320 in FIG. 3. The electronic component 420 has a second electrical property 420 that is configured to change based at least in part on a temperature of the electronic component 420. For example, in some implementations, the electronic component 420 is a thermistor (e.g., a resistor). In some such implementations, the portion 430 of the wire 410 includes a first resistance, and the thermistor 420 includes a second resistance that is greater than the first resistance.

For example, in some implementations, the second resistance of the thermistor 420 is about 2 times, 3 times, 5 times, 10 times, 15 times, 20 times, 30 times, 40 times, or 50 times of the first resistance of the portion 430 of the wire 410. Additionally, or alternatively, in some implementations, the first resistance of the portion 430 of the wire 410 is between about 5 ohms to about 5,000 ohms, such as about 1,000 ohms. In some implementations, the second resistance of the thermistor 420 is between about 5,000 ohms to about 50,000 ohms, such as about 10,000 ohms.

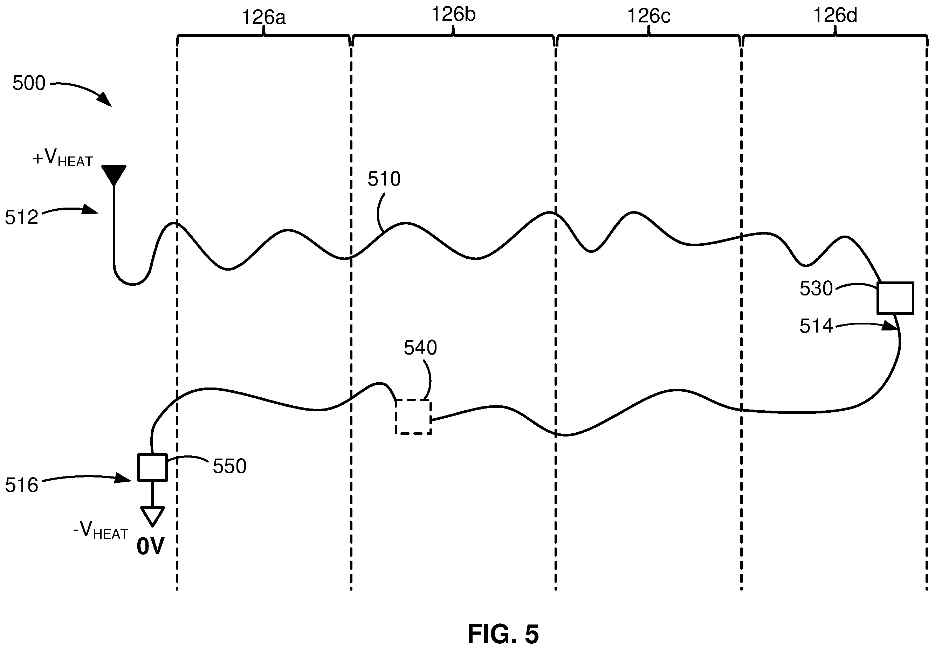

FIG. 5 depicts an electronic circuit 500, where the first electronic component of FIG. 3 is coupled to a heating wire 510, according to some implementations of the present disclosure. As shown, the electronic circuit 500 is the same as, or similar to, the electronic circuit 300 in FIG. 3, where like reference numbers are used for like elements, except that the wire 510 can be used to generate heat and is not coupled to a thermistor.

As disclosed herein, in some implementations, the respiratory device 122 (FIGS. 1-2) includes a heater to heat the water in the humidification tank 129 in order to humidify the pressurized air provided to the user. In some such implementations, the conduit 126 can include a heating element that heats the pressurized air delivered to the user. For example, the heating element can include the heating wire 510. Thus, an existing heating wire of a respiratory therapy system can be modified into the heating wire 510 of the electronic circuit 500, which is then used to generate data to determine movement associated with the heating wire 510.

According to some implementations of the present disclosure, a system for determining movement of the conduit can include multiple wires. For example, a multi-wire system can include a first electronic circuit and a second electronic circuit. The first electronic circuit is coupled to a conduit (e.g., the conduit 126), similarly to the electronic circuit 300 in FIG. 3 is coupled to its corresponding conduit. A first segment of the first electronic circuit, which corresponds to a first segment of the conduit, has an electrical resistance that is configured to change by at least a first percentage based on movement of at least a portion of the first segment of the first electronic circuit. In some implementations, the first percentage is at least 1%, at least 2%, at least 3 %, at least 4%, at least 5%, at least 6%, at least 7%, at least 8%, at least 9%, at least 10%, at least 11%, at least 12%, at least 13%, at least 14%, or at least 15%. A second segment of the first electronic circuit, which corresponds to a second segment of the conduit, has an electrical resistance that is configured to change no more than a second percentage, which is less than the first percentage based on movement of the second segment of the first electronic circuit.

The second electronic circuit is coupled to the conduit (e.g., the conduit 126), similarly to the electronic circuit 300 in FIG. 3 is coupled to its corresponding conduit. A first segment of the second electronic circuit, which corresponds to the first portion of the conduit, has an electrical resistance that is configured to change no more than the second percentage based on movement of the first segment of the second electronic circuit. A second segment of the second electronic circuit, which corresponds to the second segment of the conduit, has an electrical resistance that is configured to change by at least the first percentage based on movement of at least a portion of the second segment of the second electronic circuit.

In some implementations, first data associated with the electrical resistance of the first electronic circuit is received. Second data associated with the electrical resistance of the second electronic circuit is received. The received first data and the received second data are analyzed, to determine if (i) the electrical resistance of the first electronic circuit has changed, (ii) the electrical resistance of the second electronic circuit has changed, or (iii) both (i) and (ii). Responsive to a determination that the electrical resistance of the first electronic circuit has changed, it can be determined that the first portion of the conduit is moving or has moved. Responsive to a determination that the electrical resistance of the second electronic circuit has changed, it can be determined that the second portion of the conduit is moving or has moved.

Furthermore, as described herein, the system 100 generally can be used to generate physiological data associated with a user (e.g., a user of the respiratory therapy system 120 shown in FIG. 2) during a sleep session. The physiological data can be analyzed to generate one or more sleep-related parameters, which can include any parameter, measurement, etc. related to the user during the sleep session. The one or more sleep-related parameters that can be determined for the user 210 during the sleep session include, for example, an Apnea-Hypopnea Index (AHI) score, a sleep score, a flow signal, a respiration signal, a respiration rate, an inspiration amplitude, an expiration amplitude, an inspiration-expiration ratio, a number of events per hour, a pattern of events, a sleep stage and/or sleep state, pressure settings of the respiratory device 122, a heart rate, a heart rate variability, movement of the user 210, temperature, EEG activity, EMG activity, arousal, snoring, choking, coughing, whistling, wheezing, or a combination thereof.

Therefore, an accurate determination of sleep-wake status based on movement of the conduit would enable better calculation of residual AHI, because the physiological data when the user is not actually asleep will not be used to incorrectly impact the AHI calculation, which should only be based on flow events when the user is actually asleep. Furthermore, movement of the conduit and/or movement of the user indicated by movement of the conduit, which could confound event detection sensors (e.g. flow sensor 134), can be used to omit erroneously assigned events from the AHI calculation. Thus, according to some implementations, the present disclosure permits better management of patient therapy, by, for example, not reporting a higher AHI than necessary. Furthermore, more accurate sleep-staging based on movement of the conduit enables better sleep-staging, which in turn allows for the delivery of a better user experience and potentially boosting therapy compliance.

## Claims

1. (amended) A processor-implemented method for determining movement of a deformable conduit configured tc provide pressurized air to a user interface wearable by a patient, the method comprising:
receiving data associated with a first electrical property of a portion of an electronic circuit, the electronic circuit being coupled to the conduit and the conduit being coupled to a respiratory therapy device, wherein the first electrical property changes based at least in part on movement of the portion of the electronic circuit coupled to the conduit from a change of a structural dimension of the conduit when the conduit is coupled to the respiratory therapy device;
analyzing the received data;
determining, based at least in part on the analysis, that the first electrical property of the electronic circuit has changed; and
responsive to the determination that the first electrical property of the electronic circuit has changed, determining a frequency at which the conduit is moving or has moved.

2. The method of claim 1, wherein the portion of the electronic circuit is a conductor optionally wherein the conductor has a resistance that is configured to change by at least ten percent when moved.

3. The method of any one of claims 1 or 2, wherein the portion of the electronic circuit includes a first electronic component having the first electrical property that is configured to change based at least in part on movement of the first electronic component, and wherein the movement of the first electronic component is indicative of the movement of the portion of the electronic circuit optionally, wherein the movement of the first electronic component is indicative of a corresponding movement of at least a segment of the conduit; and/or
wherein the electronic circuit further includes a second electronic component having a second electrical property that is configured to change based at least in part on a temperature of the second electronic component; and/or
wherein the electronic circuit further includes a current sensor, the current sensor being configured to (i) measure a current flowing through the electronic circuit and (ii) generate the received data associated with the first electrical property of the portion of the electronic circuit, optionally wherein the received data is current data that is associated with the measured current flowing through the electronic circuit, and/or wherein the analysis of the received data includes identifying changes in the current flowing through the electronic circuit, and/or wherein the analysis of the received data further includes identifying a first magnitude of the identified changes in the current flowing through the electronic circuit, and wherein the identified first magnitude is associated with the movement of the first electronic component, optionally wherein the analysis of the received data further includes identifying a second magnitude of the identified changes in the current flowing through the electronic circuit, and wherein the identified second magnitude is associated with the temperature of the second electronic component.

4. The method of claim 3, wherein the analysis of the received data further includes identifying a first rate of change of a magnitude of the current flowing through the electronic circuit, and wherein the identified first rate of change is associated with the movement of the first electronic component, optionally wherein the analysis of the received data further includes identifying a second rate of change of the magnitude of the identified changes in the current flowing through the electronic circuit, wherein the identified second rate of change is associated with the temperature of the second electronic component, and wherein the identified second rate of change is different from the identified first rate of change.

5. The method of any one of claims 3 or 4, wherein the electronic circuit further includes a second electronic component positioned on the electronic circuit at a distance from the first electronic component, the second electronic component having a second electrical property that is configured to change based at least in part on movement of the second electronic component optionally wherein the method further comprises:
receiving data associated with the second electrical property of the second electronic component of the electronic circuit;
determining, based at least in part on the analysis, whether the second electrical property of the second electronic component has changed; and
responsive to the determination that the second electrical property of the second electronic component has changed, determining that the conduit is moving adjacent to the second electronic component.

6. (Amended) A system comprising:
an electronic circuit coupled to a deformable conduit providing pressurized air to a user interface wearable by a patient, a portion of the electronic circuit having a first electrical property that changes based at least in part on movement of the portion of the electronic circuit coupled to the conduit from a change of a structural dimension of the conduit when the conduit is coupled to the respiratory therapy device;
a memory storing machine-readable instructions; and
a control system including one or more processors configured to execute the machine-readable instructions to:
receive data associated with the first electrical property of the electronic circuit when the conduit delivers pressurized air;
analyze the received data;
determine, based at least in part on the analysis, that the first electrical property of the electronic circuit has changed; and
responsive to the determination that the first electrical property of the electronic circuit has changed, determine a frequency at which the conduit is moving or has moved.

7. (amended) The system of claim 6, wherein the conduit is configured to deliver pressurized air from a respiratory device to the user interface, the user interface being configured to be worn on a head of a user; and/or
wherein the portion of the electronic circuit is a conductor, optionally wherein the conductor has a resistance that is configured to change by at least ten percent when moved and/or wherein the conductor has a resistance that is configured to change by at least ten percent when stretched, compressed, bent, twisted, or any combination thereof; and/or
wherein the first electrical property of the electronic circuit is changed responsive to a change in shape, stretch, bend, or twist of the portion of the electronic circuit, wherein the shape, the stretch, the bend, or the twist of the portion of the electronic circuit is configured to change based at least in part on the movement of the portion of the electronic circuit.

8. The system of any one of claims 6 or 7, wherein the electronic circuit includes an electronic component having a second electrical property that is configured to change based at least in part on a temperature of the electronic component, optionally wherein the electronic component is a thermistor, further optionally wherein the portion of the electronic circuit includes a first resistance, and wherein the thermistor includes a second resistance that is greater than the first resistance, and still further optionally wherein the second resistance of the thermistor is about 10 times that of the first resistance of the portion of the electronic circuit and/or wherein the first resistance of the portion of the electronic circuit is about 1,000 ohms, and wherein the second resistance of the thermistor is about 10,000 ohms.

9. The system of any one of claims 6 to 8, wherein the portion of the electronic circuit includes a first electronic component having the first electrical property that is configured to change based at least in part on movement of the first electronic component, and wherein the movement of the first electronic component is indicative of the movement of the portion of the electronic circuit, optionally wherein the electronic circuit further includes a conductor, and wherein the first electronic component is electrically coupled to the conductor, further optionally wherein the conductor includes one or more electrical wires;
and/or
wherein at least a segment of the electronic circuit is physically integrated into the conduit, and/or
wherein a first segment of the electronic circuit is physically integrated into the conduit and a second segment of the electronic circuit is physically positioned within a respiratory device that is coupled to the conduit.

10. The system of claim 9, wherein the movement of the first electronic component is indicative of a corresponding movement of at least a segment of the conduit; and/or
wherein the electronic circuit further includes a second electronic component having a second electrical property that is configured to change based at least in part on a temperature of the second electronic component, optionally wherein the second electronic component is a thermistor and the second electrical property is a resistance of the thermistor.

11. The system of any one of claims 9 or 10, wherein the electronic circuit further includes a current sensor, the current sensor being configured to (i) measure a current flowing through the electronic circuit and (ii) generate the received data associated with the first electrical property of the portion of the electronic circuit, optionally wherein the received data is current data that is associated with the measured current flowing through the electronic circuit, and/or
wherein the received data is current data that is associated with the measured current flowing through the electronic circuit, wherein optionally wherein the analysis of the received data further includes identifying a first magnitude of the identified changes in the current flowing through the electronic circuit, further optionally wherein the identified first magnitude is associated with the movement of the first electronic component and/or
wherein the analysis of the received data further includes identifying a second magnitude of the identified changes in the current flowing through the electronic circuit, and wherein the identified second magnitude is associated with the temperature of the second electronic component, optionally wherein the identified second magnitude is less than the identified first magnitude.

12. The system of claim 11, wherein the analysis of the received data further includes identifying a first rate of change of a magnitude of the current flowing through the electronic circuit, optionally wherein the identified first rate of change is associated with the movement of the first electronic component and/or
wherein the analysis of the received data further includes identifying a second rate of change of the magnitude of the identified changes in the current flowing through the electronic circuit, wherein the identified second rate of change is associated with the temperature of the second electronic component, and wherein the identified second rate of change is different from the identified first rate of change.

13. The system of any one of claims 9 to 12, wherein the first electronic component includes one or more resistors, one or more semiconductors, one or more capacitors, one or more inductors, one or more ferroelectric sensors, one or more piezoelectric sensors, one or more graphene sensors, one or more optical sensors, one or more gyroscopes, one or more accelerometers, or any combination thereof;
and/or
wherein the first electronic component includes at least one of a resistor, the resistor having a resistance configured to change based at least in part on movement of the resistor, optionally wherein a standard resistance of the resistor is between about 100 ohms to about 100,000 ohms, optionally wherein a standard resistance of the resistor is about 10,000 ohms; a capacitor, the capacitor having a capacitance configured to change based at least in part on movement of the capacitor, optionally, wherein a standard capacitance of the capacitor is about 1 picofarad (pF) to 100 nanofarads (nF); or an inductor, the inductor having an inductance configured to change based at least in part on movement of the inductor, optionally, wherein a standard inductance of the inductor is about 1 nanohenry (nH) to 100 micohenrys (mH).

14. The system of any one of claims 9 to 13, further comprising the respiratory device and the user interface, the conduit being arranged between and coupled to the respiratory device and the user interface, the user interface being configured to be coupled to a face of the user to deliver the pressurized air, optionally wherein the first electronic component is positioned adjacent to the user interface; and/or
wherein the electronic circuit further includes a second electronic component positioned on the electronic circuit at a distance from the first electronic component, the second electronic component having a second electrical property that is configured to change based at least in part on movement of the second electronic component, optionally wherein the second electronic component is the same as the first electronic component, and/or
wherein the determining that the conduit is moving includes determining that the conduit is moving adjacent to the first electronic component, and/or
wherein the one or more processors are further configured to:
receive data associated with the second electrical property of the second electronic component of the electronic circuit;
determine, based at least in part on the analysis, whether the second electrical property of the second electronic component has changed; and
responsive to the determination that the second electrical property of the second electronic component has changed, determine that the conduit is moving adjacent to the second electronic component.

15. (amended) A computer program product comprising instructions which, when executed by a computer, cause the computer to carry out:
receiving first data associated with a first electrical resistance of a first electronic circuit, the first electronic circuit being coupled to a deformable conduit providing pressurized air to a user interface wearable by a patient, wherein a first segment of the first electronic circuit, which corresponds to a first segment of the conduit, has an electrical resistance that changes by at least a first percentage based on movement of at least a portion of the first segment of the first electronic circuit from a change of a structural dimension of the conduit, and
wherein a second segment of the first electronic circuit, which corresponds to a second segment of the conduit, has an electrical resistance that is configured to change no more than a second percentage that is less than the first percentage based on movement of the second segment of the first electronic circuit;
receiving second data associated with a second electrical resistance of a second electronic circuit, the second electronic circuit being coupled to the conduit, wherein a first segment of the second electronic circuit, which corresponds to the first portion of the conduit, has an electrical resistance that is configured to change no more than the second percentage based on movement of the first segment of the second electronic circuit, and wherein a second segment of the second electronic circuit, which corresponds to the second segment of the conduit, has an electrical resistance that is configured to change by at least the first percentage based on movement of at least a portion of the second segment of the second electronic circuit;
analyzing the received first data and the received second data to determine if (i) the first electrical resistance of the first electronic circuit has changed, (ii) the second electrical resistance of the second electronic circuit has changed, or (iii) both (i) and (ii) when the conduit is coupled to the respiratory device to provide air pressurized air to the conduit;
responsive to a determination that the first electrical resistance of the first electronic circuit has changed, determining that the first portion of the conduit is moving or has moved;
responsive to a determination that the second electrical resistance of the second electronic circuit has changed, determining that the second portion of the conduit is moving or has moved; and
based on the determinations of the moving of the first and second portions of the conduit, determining the frequency of movement of the conduit.

## Patentansprüche

1. Prozessorimplementiertes Verfahren zum Bestimmen einer Bewegung einer verformbaren Leitung, die so konfiguriert ist, dass sie Druckluft an eine von einem Patienten tragbare Benutzerschnittstelle liefert, wobei das Verfahren Folgendes umfasst:
Empfangen von Daten, die mit einer ersten elektrischen Eigenschaft eines Teils einer elektronischen Schaltung verknüpft sind, wobei die elektronische Schaltung mit der Leitung verbunden ist und die Leitung mit einem Beatmungsgerät verbunden ist, wobei sich die erste elektrische Eigenschaft zumindest teilweise aufgrund einer Bewegung des mit der Leitung verbundenen Teils der elektronischen Schaltung infolge einer Änderung einer strukturellen Abmessung der Leitung ändert, wenn die Leitung mit dem Beatmungsgerät verbunden ist;
Analysieren der empfangenen Daten;
Bestimmen, zumindest teilweise aufgrund der Analyse, dass sich die erste elektrische Eigenschaft der elektronischen Schaltung geändert hat; und
als Reaktion auf die Bestimmung, dass sich die erste elektrische Eigenschaft der elektronischen Schaltung geändert hat, Bestimmen einer Frequenz, mit der sich die Leitung bewegt oder bewegt hat.

2. Verfahren nach Anspruch 1, wobei es sich bei dem Teil der elektronischen Schaltung um einen Leiter handelt, wobei der Leiter optional einen Widerstand aufweist, der konfiguriert ist, sich bei Bewegung um mindestens zehn Prozent zu ändern.

3. Verfahren nach einem der Ansprüche 1 oder 2, wobei der Teil der elektronischen Schaltung ein erstes elektronisches Bauteil mit der ersten elektrischen Eigenschaft beinhaltet, die konfiguriert ist, sich zumindest teilweise aufgrund der Bewegung des ersten elektronischen Bauteils zu ändern, und wobei die Bewegung des ersten elektronischen Bauteils optional die Bewegung des Teils der elektronischen Schaltung angibt, wobei die Bewegung des ersten elektronischen Bauteils eine entsprechende Bewegung mindestens eines Teils der Leitung angibt; und/oder
wobei die elektronische Schaltung ferner ein zweites elektronisches Bauteil mit einer zweiten elektrischen Eigenschaft beinhaltet, die konfiguriert ist, sich zumindest teilweise aufgrund einer Temperatur des zweiten elektronischen Bauteils zu ändern; und/oder
wobei die elektronische Schaltung ferner einen Stromsensor beinhaltet, wobei der Stromsensor konfiguriert ist, (i) einen durch die elektronische Schaltung fließenden Strom zu messen und (ii) die empfangenen Daten erzeugt, die mit der ersten elektrischen Eigenschaft des Teils der elektronischen Schaltung verknüpft sind, wobei es sich bei den empfangenen Daten optional um Stromdaten handelt, die mit dem gemessenen Strom verknüpft sind, der durch die elektronische Schaltung fließt, und/oder wobei die Analyse der empfangenen Daten Identifizieren von Änderungen des durch die elektronische Schaltung fließenden Stroms beinhaltet, und/oder wobei die Analyse der empfangenen Daten ferner Identifizieren einer ersten Größe der identifizierten Änderungen des durch die elektronische Schaltung fließenden Stroms beinhaltet und wobei die identifizierte erste Größe mit der Bewegung des ersten elektronischen Bauteils verknüpft ist, wobei die Analyse der empfangenen Daten optional ferner Identifizieren einer zweiten Größe der identifizierten Änderungen des durch die elektronische Schaltung fließenden Stroms beinhaltet und wobei die identifizierte zweite Größe mit der Temperatur des zweiten elektronischen Bauteils verknüpft ist.

4. Verfahren nach Anspruch 3, wobei die Analyse der empfangenen Daten ferner Identifizieren einer ersten Änderungsrate einer Größe des durch die elektronische Schaltung fließenden Stroms beinhaltet und wobei die identifizierte erste Änderungsrate mit der Bewegung des ersten elektronischen Bauteils verknüpft ist, wobei die Analyse der empfangenen Daten ferner optional Identifizieren einer zweiten Änderungsrate der Größe der identifizierten Änderungen des durch die elektronische Schaltung fließenden Stroms beinhaltet, wobei die identifizierte zweite Änderungsrate mit der Temperatur des zweiten elektronischen Bauteils verknüpft ist und wobei sich die identifizierte zweite Änderungsrate von der identifizierten ersten Änderungsrate unterscheidet.

5. Verfahren nach einem der Ansprüche 3 oder 4, wobei die elektronische Schaltung ferner ein zweites elektronisches Bauteil beinhaltet, das auf der elektronischen Schaltung in einem Abstand vom ersten elektronischen Bauteil positioniert ist, wobei das zweite elektronische Bauteil eine zweite elektrische Eigenschaft aufweist, die konfiguriert ist, sich zumindest teilweise aufgrund einer Bewegung des zweiten elektronischen Bauteils zu ändern; wobei das Verfahren optional ferner umfasst:
Empfangen von Daten, die mit der zweiten elektrischen Eigenschaft des zweiten elektronischen Bauteils der elektronischen Schaltung verknüpft sind;
Bestimmen, zumindest teilweise aufgrund der Analyse, ob sich die zweite elektrische Eigenschaft des zweiten elektronischen Bauteils geändert hat; und
als Reaktion auf die Bestimmung, dass sich die zweite elektrische Eigenschaft des zweiten elektronischen Bauteils geändert hat, Bestimmen, dass sich die Leitung neben dem zweiten elektronischen Bauteil bewegt.

6. System, umfassend:
eine elektronische Schaltung, die mit einer verformbaren Leitung verbunden ist und Druckluft an eine vom Patienten tragbare Benutzerschnittstelle liefert, wobei ein Teil der elektronischen Schaltung eine erste elektrische Eigenschaft aufweist, die sich zumindest teilweise aufgrund einer Bewegung des mit der Leitung verbundenen Teils der elektronischen Schaltung infolge einer Änderung einer strukturellen Abmessung der Leitung ändert, wenn die Leitung mit dem Beatmungsgerät verbunden ist;
einen Speicher, der maschinenlesbare Anweisungen speichert; und
ein Steuerungssystem, das einen oder mehrere Prozessoren beinhaltet, die konfiguriert sind, die maschinenlesbaren Anweisungen auszuführen, um:
Daten zu empfangen, die mit der ersten elektrischen Eigenschaft der elektronischen Schaltung verknüpft sind, wenn die Leitung Druckluft abgibt;
die empfangenen Daten zu analysieren;
zumindest teilweise aufgrund der Analyse zu bestimmen, dass sich die erste elektrische Eigenschaft der elektronischen Schaltung geändert hat; und
als Reaktion auf die Bestimmung, dass sich die erste elektrische Eigenschaft der elektronischen Schaltung geändert hat, eine Frequenz zu bestimmen, mit der sich die Leitung bewegt oder bewegt hat.

7. System nach Anspruch 6, wobei die Leitung konfiguriert ist, Druckluft von einem Beatmungsgerät an die Benutzerschnittstelle zu liefern, wobei die Benutzerschnittstelle so konfiguriert ist, dass sie auf einem Kopf eines Benutzers getragen wird; und/oder
wobei der Teil der elektronischen Schaltung ein Leiter ist, wobei der Leiter optional einen Widerstand aufweist, der konfiguriert ist, sich bei Bewegung um mindestens zehn Prozent zu ändern, und/oder wobei der Leiter einen Widerstand aufweist, der konfiguriert ist, sich bei Dehnung, Stauchung, Biegung, Verdrehung oder einer beliebigen Kombination davon um mindestens zehn Prozent zu ändern; und/oder
wobei die erste elektrische Eigenschaft der elektronischen Schaltung als Reaktion auf eine Änderung in Form, Dehnung, Biegung oder Verdrehung des Teils der elektronischen Schaltung geändert wird, wobei die Form, die Dehnung, die Biegung oder die Verdrehung des Teils der elektronischen Schaltung konfiguriert ist, sich zumindest teilweise aufgrund der Bewegung des Teils der elektronischen Schaltung zu ändern.

8. System nach einem der Ansprüche 6 oder 7, wobei die elektronische Schaltung ein elektronisches Bauteil mit einer zweiten elektrischen Eigenschaft beinhaltet, die so konfiguriert ist, dass sie sich zumindest teilweise aufgrund einer Temperatur des elektronischen Bauteils ändert, wobei es sich bei dem elektronischen Bauteil optional um einen Thermistor handelt, wobei der Teil der elektronischen Schaltung optional ferner einen ersten Widerstand beinhaltet und wobei der Thermistor einen zweiten Widerstand beinhaltet, der größer als der erste Widerstand ist, und wobei der zweite Widerstand des Thermistors optional noch ferner etwa das Zehnfache des ersten Widerstands des Teils der elektronischen Schaltung beträgt und/oder wobei der erste Widerstand des Teils der elektronischen Schaltung etwa 1.000 Ohm beträgt und wobei der zweite Widerstand des Thermistors etwa 10.000 Ohm beträgt.

9. System nach einem der Ansprüche 6 bis 8, wobei der Teil der elektronischen Schaltung ein erstes elektronisches Bauteil mit der ersten elektrischen Eigenschaft beinhaltet, die konfiguriert ist, sich zumindest teilweise aufgrund der Bewegung des ersten elektronischen Bauteils zu ändern, und wobei die Bewegung des ersten elektronischen Bauteils die Bewegung des Teils der elektronischen Schaltung angibt, wobei optional die elektronische Schaltung ferner einen Leiter beinhaltet und wobei das erste elektronische Bauteil elektrisch mit dem Leiter verbunden ist, wobei optional der Leiter ferner einen oder mehrere elektrische Drähte beinhaltet;
und/oder
wobei mindestens ein Teil der elektronischen Schaltung physisch in die Leitung integriert ist, und/oder
wobei ein erstes Segment der elektronischen Schaltung physisch in die Leitung integriert ist und ein zweites Segment der elektronischen Schaltung physisch in einem Beatmungsgerät positioniert ist, das mit der Leitung verbunden ist.

10. System nach Anspruch 9, wobei die Bewegung des ersten elektronischen Bauteils eine entsprechende Bewegung mindestens eines Segments der Leitung angibt; und/oder
wobei die elektronische Schaltung ferner ein zweites elektronisches Bauteil mit einer zweiten elektrischen Eigenschaft beinhaltet, die konfiguriert ist, sich zumindest teilweise aufgrund einer Temperatur des zweiten elektronischen Bauteils zu ändern, wobei es sich bei dem zweiten elektronischen Bauteil optional um einen Thermistor und bei der zweiten elektrischen Eigenschaft um einen Widerstand des Thermistors handelt.

11. System nach einem der Ansprüche 9 oder 10, wobei die elektronische Schaltung ferner einen Stromsensor beinhaltet, wobei der Stromsensor konfiguriert ist, (i) einen durch die elektronische Schaltung fließenden Strom misst und (ii) die empfangenen Daten zu erzeugen, die mit der ersten elektrischen Eigenschaft des Teils der elektronischen Schaltung verknüpft sind, wobei es sich bei den empfangenen Daten optional um Stromdaten handelt, die mit dem gemessenen Strom verknüpft sind, der durch die elektronische Schaltung fließt, und/oder
wobei es sich bei den empfangenen Daten um Stromdaten handelt, die mit dem gemessenen Strom verknüpft sind, der durch die elektronische Schaltung fließt, wobei die Analyse der empfangenen Daten optional ferner Identifizieren einer ersten Größe der identifizierten Änderungen des durch die elektronische Schaltung fließenden Stroms beinhaltet, wobei die identifizierte erste Größe optional ferner mit der Bewegung des ersten elektronischen Bauteils verknüpft ist, und/oder
wobei die Analyse der empfangenen Daten ferner Identifizieren einer zweiten Größe der identifizierten Änderungen des durch die elektronische Schaltung fließenden Stroms beinhaltet und wobei die identifizierte zweite Größe mit der Temperatur des zweiten elektronischen Bauteils verknüpft ist, wobei die identifizierte zweite Größe optional kleiner als die identifizierte erste Größe ist.

12. System nach Anspruch 11, wobei die Analyse der empfangenen Daten ferner Identifizieren einer ersten Änderungsrate einer Größe des durch die elektronische Schaltung fließenden Stroms beinhaltet, wobei die identifizierte erste Änderungsrate optional mit der Bewegung des ersten elektronischen Bauteils verknüpft ist, und/oder
wobei die Analyse der empfangenen Daten ferner Identifizieren einer zweiten Änderungsrate der Größe der identifizierten Änderungen des durch die elektronische Schaltung fließenden Stroms beinhaltet, wobei die identifizierte zweite Änderungsrate mit der Temperatur des zweiten elektronischen Bauteils verknüpft ist und wobei die identifizierte zweite Änderungsrate sich von der identifizierten ersten Änderungsrate unterscheidet.

13. System nach einem der Ansprüche 9 bis 12, wobei das erste elektronische Bauteil einen oder mehrere Widerstände, einen oder mehrere Halbleiter, einen oder mehrere Kondensatoren, eine oder mehrere Induktoren, einen oder mehrere ferroelektrische Sensoren, einen oder mehrere piezoelektrische Sensoren, einen oder mehrere Graphensensoren, einen oder mehrere optische Sensoren, ein oder mehrere Gyroskope, einen oder mehrere Beschleunigungsmesser oder eine beliebige Kombination davon beinhaltet;
und/oder
wobei das erste elektronische Bauteil mindestens eines von einem Widerstand, wobei der Widerstand einen Widerstandswert aufweist, der konfiguriert ist, sich zumindest teilweise aufgrund einer Bewegung des Widerstands zu ändern, wobei ein Standardwiderstand des Widerstands optional zwischen etwa 100 Ohm und etwa 100.000 Ohm liegt, wobei ein Standardwiderstand des Widerstands optional etwa 10.000 Ohm beträgt; einem Kondensator, wobei der Kondensator eine Kapazität aufweist, die konfiguriert ist, sich zumindest teilweise aufgrund einer Bewegung des Kondensators zu ändern, wobei eine Standardkapazität des Kondensators optional etwa 1 Pikofarad (pF) bis 100 Nanofarad (nF) beträgt; oder einem Induktor, wobei der Induktor eine Induktivität aufweist, die sich zumindest teilweise aufgrund einer Bewegung des Induktors ändert, wobei eine Standardinduktivität des Induktors optional etwa 1 Nanohenry (nH) bis 100 Mikrohenry (mH) beträgt, beinhaltet.

14. System nach einem der Ansprüche 9 bis 13, ferner umfassend das Beatmungsgerät und die Benutzerschnittstelle, wobei die Leitung zwischen dem Beatmungsgerät und der Benutzerschnittstelle angeordnet und mit diesen verbunden ist, wobei die Benutzerschnittstelle so konfiguriert ist, dass sie mit einem Gesicht des Benutzers verbunden wird, um die Druckluft zu liefern, wobei optional das erste elektronische Bauteil neben der Benutzerschnittstelle positioniert ist; und/oder
wobei die elektronische Schaltung ferner ein zweites elektronisches Bauteil beinhaltet, das auf der elektronischen Schaltung in einem Abstand vom ersten elektronischen Bauteil positioniert ist, wobei das zweite elektronische Bauteil eine zweite elektrische Eigenschaft aufweist, die konfiguriert ist, sich zumindest teilweise aufgrund einer Bewegung des zweiten elektronischen Bauteils zu ändern, wobei das zweite elektronische Bauteil optional mit dem ersten elektronischen Bauteil identisch ist, und/oder
wobei das Bestimmen, dass sich die Leitung bewegt, Bestimmen beinhaltet, dass sich die Leitung neben dem ersten elektronischen Bauteil bewegt, und/oder
wobei der eine oder die mehreren Prozessoren ferner konfiguriert sind:
Daten zu empfangen, die mit der zweiten elektrischen Eigenschaft des zweiten elektronischen Bauteils der elektronischen Schaltung verknüpft sind;
zumindest teilweise aufgrund der Analyse zu bestimmen, ob sich die zweite elektrische Eigenschaft des zweiten elektronischen Bauteils geändert hat; und
als Reaktion auf die Bestimmung, dass sich die zweite elektrische Eigenschaft des zweiten elektronischen Bauteils geändert hat, zu bestimmen, dass sich die Leitung neben dem zweiten elektronischen Bauteil bewegt.

15. Computerprogrammprodukt, das Anweisungen umfasst, die, wenn sie von einem Computer ausgeführt werden, bewirken, dass der Computer Folgendes ausführt:
Empfangen erster Daten, die mit einem ersten elektrischen Widerstand einer ersten elektronischen Schaltung verknüpft sind, wobei die erste elektronische Schaltung mit einer verformbaren Leitung verbunden ist, die Druckluft zu einer vom Patienten tragbaren Benutzerschnittstelle liefert, wobei ein erstes Segment der ersten elektronischen Schaltung, das einem ersten Segment der Leitung entspricht, einen elektrischen Widerstand aufweist, der sich aufgrund einer Bewegung mindestens eines Teils des ersten Segments der ersten elektronischen Schaltung infolge einer Änderung einer strukturellen Abmessung der Leitung um mindestens einen ersten Prozentsatz ändert, und
wobei ein zweites Segment der ersten elektronischen Schaltung, das einem zweiten Segment der Leitung entspricht, einen elektrischen Widerstand aufweist, der konfiguriert ist, sich aufgrund einer Bewegung des zweiten Segments der ersten elektronischen Schaltung um höchstens einen zweiten Prozentsatz zu ändern, der kleiner ist als der erste Prozentsatz;
Empfangen von zweiten Daten, die mit einem zweiten elektrischen Widerstand einer zweiten elektronischen Schaltung verknüpft sind, wobei die zweite elektronische Schaltung mit der Leitung verbunden ist, wobei ein erstes Segment der zweiten elektronischen Schaltung, das dem ersten Teil der Leitung entspricht, einen elektrischen Widerstand aufweist, der konfiguriert ist, sich aufgrund einer Bewegung des ersten Segments der zweiten elektronischen Schaltung um höchstens den zweiten Prozentsatz zu ändern, und wobei ein zweites Segment der zweiten elektronischen Schaltung, das dem zweiten Segment der Leitung entspricht, einen elektrischen Widerstand aufweist, der konfiguriert ist, sich aufgrund einer Bewegung mindestens eines Teils des zweiten Segments der zweiten elektronischen Schaltung um mindestens den ersten Prozentsatz zu ändern;
Analysieren der empfangenen ersten Daten und der empfangenen zweiten Daten, um zu bestimmen, ob (i) sich der erste elektrische Widerstand der ersten elektronischen Schaltung geändert hat, (ii) sich der zweite elektrische Widerstand der zweiten elektronischen Schaltung geändert hat, oder (iii) sowohl (i) als auch (ii) zu bestimmen, wenn die Leitung mit dem Beatmungsgerät verbunden ist, um der Leitung Druckluft zu liefern;
als Reaktion auf eine Bestimmung, dass sich der erste elektrische Widerstand der ersten elektronischen Schaltung geändert hat, Bestimmen, dass sich der erste Teil der Leitung bewegt oder bewegt hat;
als Reaktion auf eine Bestimmung, dass sich der zweite elektrische Widerstand der zweiten elektronischen Schaltung geändert hat, Bestimmen, dass sich der zweite Teil der Leitung bewegt oder bewegt hat; und
aufgrund der Bestimmungen der Bewegung des ersten und zweiten Teils der Leitung Bestimmen der Bewegungsfrequenz der Leitung.

## Revendications

1. Procédé mis en œuvre par processeur pour déterminer le mouvement d'un conduit déformable configuré pour fournir de l'air comprimé à une interface utilisateur portable par un patient, le procédé comprenant :
la réception de données associées à une première propriété électrique d'une portion d'un circuit électronique, le circuit électronique étant couplé au conduit et le conduit étant couplé à un dispositif de thérapie respiratoire, dans lequel la première propriété électrique change au moins en partie en raison du mouvement de la portion du circuit électronique couplée au conduit du fait d'un changement d'une dimension structurelle du conduit lorsque celui-ci est couplé au dispositif de thérapie respiratoire ;
l'analyse des données reçues ;
la détermination, en se basant au moins en partie sur l'analyse, que la première propriété électrique du circuit électronique a changé ; et
en réponse à la constatation que la première propriété électrique du circuit électronique a changé, la détermination d'une fréquence à laquelle le conduit se déplace ou s'est déplacé.

2. Procédé selon la revendication 1, dans lequel la portion du circuit électronique est un conducteur dans lequel, éventuellement, le conducteur présente une résistance configurée pour changer d'au moins dix pour cent lorsqu'il est déplacé.

3. Procédé selon l'une quelconque des revendications 1 ou 2, dans lequel la portion du circuit électronique inclut un premier composant électronique présentant la première propriété électrique qui est configurée pour changer au moins en partie en fonction du mouvement du premier composant électronique, et dans lequel le mouvement du premier composant électronique est indicatif du mouvement de la portion du circuit électronique, éventuellement, dans lequel le mouvement du premier composant électronique est indicatif d'un mouvement correspondant d'au moins un segment du conduit ; et/ou
dans lequel le circuit électronique inclut en outre un deuxième composant électronique présentant une deuxième propriété électrique configurée pour changer au moins en partie en fonction de la température du deuxième composant électronique ; et/ou
dans lequel le circuit électronique inclut en outre un capteur de courant, ce capteur étant configuré pour (i) mesurer un courant circulant dans le circuit électronique et (ii) générer les données reçues associées à la première propriété électrique de la portion du circuit électronique, dans lequel les données reçues sont éventuellement des données de courant associées au courant mesuré circulant dans le circuit électronique et/ou dans lequel l'analyse des données reçues inclut l'identification des changements du courant circulant dans le circuit électronique, et/ou dans lequel l'analyse des données reçues inclut en outre l'identification d'une première amplitude des variations identifiées du courant circulant dans le circuit électronique, et dans lequel la première amplitude identifiée est associée au mouvement du premier composant électronique, et, éventuellement, dans lequel l'analyse des données reçues inclut en outre l'identification d'une deuxième amplitude des variations identifiées du courant circulant dans le circuit électronique, et dans lequel la deuxième amplitude identifiée est associée à la température du deuxième composant électronique.

4. Procédé selon la revendication 3, dans lequel l'analyse des données reçues inclut en outre l'identification d'un premier taux de variation de l'amplitude du courant circulant dans le circuit électronique, et dans lequel le premier taux de variation identifié est associé au mouvement du premier composant électronique, et, de manière optionnelle, dans lequel l'analyse des données reçues inclut en outre l'identification d'un deuxième taux de variation de l'amplitude des variations identifiées du courant circulant dans le circuit électronique, dans lequel le deuxième taux de variation identifié est associé à la température du deuxième composant électronique, et dans lequel le deuxième taux de variation identifié est différent du premier taux de variation identifié.

5. Procédé selon l'une quelconque des revendications 3 ou 4, dans lequel le circuit électronique inclut en outre un deuxième composant électronique positionné sur le circuit électronique à une distance du premier composant électronique, le deuxième composant électronique présentant une deuxième propriété électrique configurée pour changer au moins en partie en fonction du mouvement du deuxième composant électronique, dans lequel le procédé comprend en outre :
la réception des données associées à la deuxième propriété électrique du deuxième composant électronique du circuit électronique ;
la détermination, en se basant au moins en partie sur l'analyse, si la deuxième propriété électrique du deuxième composant électronique a changé ; et
en réponse à la constatation que la deuxième propriété électrique du deuxième composant électronique a changé, la constatation que le conduit se déplace à proximité du deuxième composant électronique.

6. Système comprenant :
un circuit électronique couplé à un conduit déformable fournissant de l'air comprimé à une interface utilisateur portable par un patient, une portion du circuit électronique présentant une première propriété électrique qui change au moins en partie en fonction du mouvement de la portion du circuit électronique couplée au conduit à partir d'un changement d'une dimension structurelle du conduit lorsque celui-ci est couplé au dispositif de thérapie respiratoire ;
un dispositif de mémoire stockant des instructions lisibles par machine ; et
un système de commande incluant un ou plusieurs processeurs sont en outre configurés pour exécuter les instructions lisibles par machine pour :
recevoir des données associées à la première propriété électrique du circuit électronique lorsque le conduit délivre de l'air comprimé ;
analyser les données reçues ;
déterminer, en se basant au moins en partie sur l'analyse, que la première propriété électrique du circuit électronique a changé ; et
en réponse à la constatation que la première propriété électrique du circuit électronique a changé, déterminer une fréquence à laquelle le conduit se déplace ou s'est déplacé.

7. Système selon la revendication 6, dans lequel le conduit est configuré pour acheminer de l'air sous pression d'un dispositif respiratoire vers l'interface utilisateur, cette dernière étant configurée pour être portée sur la tête d'un utilisateur ; et/ou
dans lequel la portion du circuit électronique est un conducteur, éventuellement dans lequel le conducteur possède une résistance configurée pour varier d'au moins dix pour cent lorsqu'elle est déplacée et/ou dans lequel le conducteur possède une résistance configurée pour varier d'au moins dix pour cent lorsqu'elle est étirée, comprimée, pliée, tordue ou toute combinaison de ces opérations ; et/ou
dans lequel la première propriété électrique du circuit électronique est modifiée en réponse à un changement de forme, d'étirement, de courbure ou de torsion de la portion du circuit électronique, dans lequel la forme, l'étirement, la courbure ou la torsion de la portion du circuit électronique sont configurées pour changer au moins en partie en fonction du mouvement de la portion du circuit électronique.

8. Système selon l'une quelconque des revendications 6 ou 7, dans lequel le circuit électronique inclut un composant électronique présentant une deuxième propriété électrique configurée pour varier au moins en partie en fonction de la température du composant électronique, dans lequel le composant électronique est éventuellement une thermistance ; de plus, éventuellement, dans lequel la portion de circuit électronique inclut une première résistance, et dans lequel la thermistance inclut une deuxième résistance supérieure à la première, et de plus, éventuellement, la deuxième résistance de la thermistance est environ 10 fois supérieure à la première résistance de la portion de circuit électronique et/ou dans lequel la première résistance de la portion du circuit électronique est d'environ 1 000 ohms, et dans lequel la deuxième résistance de la thermistance est d'environ 10 000 ohms.

9. Système selon l'une quelconque des revendications 6 à 8, dans lequel la portion du circuit électronique inclut un premier composant électronique présentant la première propriété électrique qui est configurée pour changer au moins en partie en fonction du mouvement du premier composant électronique, et dans lequel le mouvement du premier composant électronique est indicatif du mouvement de la portion du circuit électronique, éventuellement dans lequel le circuit électronique inclut en outre un conducteur, et dans lequel le premier composant électronique est couplé électriquement au conducteur, éventuellement dans lequel le conducteur inclut en outre un ou plusieurs fils électriques ;
et/ou
dans lequel au moins un segment du circuit électronique est physiquement intégré dans le conduit, et/ou
dans lequel un premier segment du circuit électronique est physiquement intégré dans le conduit et un deuxième segment du circuit électronique est physiquement positionné à l'intérieur d'un dispositif respiratoire qui est couplé au conduit.

10. Système selon la revendication 9, dans lequel le mouvement du premier composant électronique est indicatif d'un mouvement correspondant d'au moins un segment du conduit ; et/ou
dans lequel le circuit électronique inclut en outre un deuxième composant électronique présentant une deuxième propriété électrique configurée pour changer au moins en partie en fonction de la température du deuxième composant électronique, dans lequel, éventuellement, le deuxième composant électronique est une thermistance et la deuxième propriété électrique est une résistance de la thermistance.

11. Système selon l'une quelconque des revendications 9 ou 10, dans lequel le circuit électronique inclut en outre un capteur de courant, ce capteur de courant étant configuré pour (i) mesurer un courant circulant dans le circuit électronique et (ii) générer les données reçues associées à la première propriété électrique de la portion du circuit électronique, dans lequel les données reçues sont éventuellement des données de courant associées au courant mesuré circulant dans le circuit électronique, et/ou
dans lequel les données reçues sont des données de courant associées au courant mesuré circulant dans le circuit électronique, et, dans lequel, éventuellement, l'analyse des données reçues inclut en outre l'identification d'une première amplitude de la variation du courant circulant dans le circuit électronique, dans lequel en outre la première amplitude est éventuellement associée au mouvement du premier composant électronique, et/ou
dans lequel l'analyse des données reçues inclut en outre l'identification d'une deuxième amplitude des variations identifiées du courant circulant dans le circuit électronique, et dans lequel la deuxième amplitude identifiée est associée à la température du deuxième composant électronique, éventuellement dans lequel la deuxième amplitude identifiée est inférieure à la première amplitude identifiée.

12. Système selon la revendication 11, dans lequel l'analyse des données reçues inclut en outre l'identification d'une première vitesse de variation de l'amplitude du courant circulant dans le circuit électronique, dans lequel cette première vitesse de variation est éventuellement associée au mouvement du premier composant électronique, et/ou
dans lequel l'analyse des données reçues inclut en outre l'identification d'un deuxième taux de variation de l'amplitude des variations identifiées du courant circulant dans le circuit électronique, dans lequel le deuxième taux de variation identifié est associé à la température du deuxième composant électronique, et dans lequel le deuxième taux de variation identifié étant différent du premier taux de variation identifié.

13. Système selon l'une quelconque des revendications 9 à 12, dans lequel le premier composant électronique inclut une ou plusieurs résistances, un ou plusieurs semiconducteurs, un ou plusieurs condensateurs, une ou plusieurs inductances, un ou plusieurs capteurs ferroélectriques, un ou plusieurs capteurs piézoélectriques, un ou plusieurs capteurs au graphène, un ou plusieurs capteurs optiques, un ou plusieurs gyroscopes, un ou plusieurs accéléromètres, ou toute combinaison de ceux-ci ;
et/ou
dans lequel le premier composant électronique inclut au moins un élément parmi les suivants : une résistance, la résistance présentant une valeur configurée pour varier en fonction, au moins en partie, du mouvement de la résistance, dans lequel la valeur standard de la résistance est comprise entre environ 100 ohms et environ 100 000 ohms, dans lequel la valeur standard de la résistance est éventuellement d'environ 10 000 ohms ; un condensateur, le condensateur présentant une capacité configurée pour varier en fonction, au moins en partie, du mouvement du condensateur, dans lequel la valeur standard du condensateur est comprise entre environ 1 picofarad (pF) et 100 nanofarads (nF) ; ou un inducteur, l'inductance de l'inducteur étant configurée pour varier en fonction, au moins en partie, du mouvement de l'inducteur, dans lequel la valeur standard de l'inductance de l'inducteur est comprise entre environ 1 nanohenry (nH) et 100 microhenrys (mH).

14. Système selon l'une quelconque des revendications 9 à 13, comprenant en outre le dispositif respiratoire et l'interface utilisateur, le conduit étant disposé entre et couplé au dispositif respiratoire et à l'interface utilisateur, l'interface utilisateur étant configurée pour être couplée au visage de l'utilisateur pour délivrer l'air comprimé, dans lequel le premier composant électronique est éventuellement positionné de manière adjacente à l'interface utilisateur ; et/ou
dans lequel le circuit électronique inclut en outre un deuxième composant électronique positionné sur le circuit électronique à une distance du premier composant électronique, le deuxième composant électronique présentant une deuxième propriété électrique configurée pour changer au moins en partie en fonction du mouvement du deuxième composant électronique, de préférence dans lequel le deuxième composant électronique est le même que le premier composant électronique, et/ou
dans lequel la détermination du déplacement du conduit inclut la détermination du déplacement du conduit à proximité du premier composant électronique, et/ou
dans lequel le ou les processeurs sont en outre configurés pour :
recevoir des données associées à la deuxième propriété électrique du deuxième composant électronique du circuit électronique ;
déterminer, en se basant au moins en partie sur l'analyse, si la deuxième propriété électrique du deuxième composant électronique a changé ; et
en réponse à la détermination que la deuxième propriété électrique du deuxième composant électronique a changé, déterminer que le conduit se déplace à proximité du deuxième composant électronique.

15. Produit de programme informatique comprenant des instructions qui, lorsqu'elles sont exécutées par un ordinateur, amènent l'ordinateur à réaliser les opérations suivantes :
réception de premières données associées à une première résistance électrique d'un premier circuit électronique, ce premier circuit électronique étant couplé à un conduit déformable fournissant de l'air comprimé à une interface utilisateur portable par un patient, dans lequel un premier segment du premier circuit électronique, qui correspond à un premier segment du conduit, présente une résistance électrique qui varie d'au moins un premier pourcentage en fonction du mouvement d'au moins une portion du premier segment du premier circuit électronique suite à une modification d'une dimension structurelle du conduit, et
dans lequel un deuxième segment du premier circuit électronique, qui correspond à un deuxième segment du conduit, présente une résistance électrique configurée pour ne pas varier de plus d'un deuxième pourcentage inférieur au premier pourcentage en fonction du mouvement du deuxième segment du premier circuit électronique ;
réception de deuxièmes données associées à une deuxième résistance électrique d'un deuxième circuit électronique, le deuxième circuit électronique étant couplé au conduit, dans lequel un premier segment du deuxième circuit électronique, qui correspond à la première portion du conduit, présente une résistance électrique configurée pour ne pas varier de plus du deuxième pourcentage en fonction du mouvement du premier segment du deuxième circuit électronique, et dans lequel un deuxième segment du deuxième circuit électronique, qui correspond au deuxième segment du conduit, présente une résistance électrique configurée pour varier d'au moins le premier pourcentage en fonction du mouvement d'au moins une portion du deuxième segment du deuxième circuit électronique ;
analyse des premières données reçues et des deuxièmes données reçues pour déterminer si (i) la première résistance électrique du premier circuit électronique a changé, (ii) la deuxième résistance électrique du deuxième circuit électronique a changé, ou (iii) les deux (i) et (ii) lorsque le conduit est couplé au dispositif respiratoire pour fournir de l'air comprimé au conduit ;
en réponse à une détermination que la première résistance électrique du premier circuit électronique a changé, la détermination que la première portion du conduit est en mouvement ou s'est déplacée ;
en réponse à une détermination que la deuxième résistance électrique du deuxième circuit électronique a changé, la détermination que la deuxième portion du conduit est en mouvement ou s'est déplacée ; et
en se basant sur les déterminations du déplacement des première et deuxième portions du conduit, en déterminant la fréquence de déplacement du conduit.
